(19) **Europäisches Patentamt European Patent Office Office européen des brevets**

(11) **EP 4 034 058 B1**

(12) **EUROPEAN PATENT SPECIFICATION**

(45) Date of publication and mention of the grant of the patent:
**09.07.2025 Bulletin 2025/28**

(21) Application number: **20786444.8**

(22) Date of filing: **25.09.2020**

(51) International Patent Classification (IPC):
*A61F 13/00* (2024.01)     *G01M 99/00* (2011.01)
*G01R 31/52* (2020.01)

(52) Cooperative Patent Classification (CPC):
**G01M 99/005**

(86) International application number:
**PCT/DK2020/050262**

(87) International publication number:
**WO 2021/063463 (08.04.2021 Gazette 2021/14)**

(54) **APPLIANCE INTERFACE, SYSTEM, AND METHOD FOR LIQUID INGRESS DETECTION IN THE APPLIANCE INTERFACE**

GERÄTESCHNITTSTELLE, SYSTEM UND VERFAHREN ZUR FLÜSSIGKEITSEINTRITTSERKENNUNG IN DIE GERÄTESCHNITTSTELLE

INTERFACE D'APPAREIL, SYSTÈME ET PROCÉDÉ DE DÉTECTION D'ENTRÉE DE LIQUIDE DANS L'INTERFACE D'APPAREIL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.09.2019 DK PA201970587**

(43) Date of publication of application:
**03.08.2022 Bulletin 2022/31**

(73) Proprietor: **Coloplast A/S**
**3050 Humlebaek (DK)**

(72) Inventors:
  • **OLSEN, Jesper Kenneth**
    **3460 Birkeroed (DK)**
  • **SPEIERMANN, Finn**
    **2830 Virum (DK)**
  • **MOLZEN, Lars**
    **2800 Kongens Lyngby (DK)**
  • **HANSEN, Jais Ask**
    **3630 Jaegerspris (DK)**
  • **SUND, Kamilla Grove**
    **3060 Espergaerde (DK)**
  • **EMBORG, Jonas**
    **3600 Frederikssund (DK)**

(56) References cited:
**WO-A1-2007/098762     US-A1- 2009 314 101
US-A1- 2019 079 037**

## Description

The present disclosure relates to an electronic system for detecting presence of liquid in an appliance interface. In particular, the present disclosure relates to an electronic system comprising an appliance interface and an electronic device couplable to such appliance interface. Further, the present disclosure relates a method for detecting presence of liquid in an appliance interface, and an appliance interface as such. Further, the present disclosure relates to a medical device comprising an electronic system for detecting presence of liquid in an appliance interface.

## Background

US 2019/079037 A1 relates to an electronic device comprising a connector including a plurality of contacts and a liquid detection module coupled to at least one contact in the plurality of contacts.
WO 2007/098762 A1 relates to a method and dressing for detecting detachment of the dressing, which is applied to a surface of an at least partly electrically conductive object. The dressing comprises an adhesive and at least two electrodes arranged in a distance from the electrically conductive object. Changes in capacitance are detected, and an alarm is activated when the changes of the capacitance reach a predetermined value.
US 2009/314101 A1 relates to a method, device and system for determining a flow rate of an excretion stream within an excretion collection assembly.

## Brief description of the drawings

The accompanying drawings are included to provide a further understanding of embodiments and are incorporated into and a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.

Fig. 1 illustrates an electronic system comprising an electronic device and an appliance interface according to an embodiment of the invention,

Fig. 2A illustrates a top view of an appliance interface according to an embodiment of the invention,

Fig. 2B illustrates a top view of an appliance interface according to an embodiment of the invention with a presence of liquid,

Fig. 3A illustrates an exemplary cross-sectional view of an appliance interface according to an embodiment of the invention,

Fig. 3B illustrates an exemplary cross-sectional view of an appliance interface according to an embodiment of the invention with a presence of liquid,

Fig. 4A illustrates a top view of an appliance interface according to an embodiment of the invention,

Fig. 4B illustrates a top view of an appliance interface according to an embodiment of the invention with a presence of liquid,

Fig. 5A illustrates a top view of an appliance interface according to an embodiment of the invention,

Fig. 5B illustrates a top view of an appliance interface according to an embodiment of the invention with a presence of liquid,

Fig. 6A illustrates a sine waveform,

Fig. 6B illustrates a square waveform,

Fig. 6C illustrates a triangular waveform,

Fig. 6D illustrates a sawtooth waveform,

Fig. 7 illustrates a top view of an appliance interface according to an embodiment of the invention,

Fig. 8A illustrates a top view of an appliance interface according to an embodiment of the invention,

Fig. 8B illustrates a cross-sectional view of an appliance interface according to an embodiment of the invention,

Fig. 9A illustrates a top view of an appliance interface according to an embodiment of the invention with a presence of liquid,

Fig. 9B illustrates a cross-sectional view of an appliance interface according to an embodiment of the invention with a presence of liquid,

Fig. 10 illustrates a perspective schematic view of an electronic system according to an embodiment of the invention,

Fig. 11 illustrates an exemplary sensor assembly comprising an electronic system according to an embodiment of the invention, and

Fig. 12 illustrates a close-up view of an appliance interface according to an embodiment of the invention.

## Detailed description

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

The use of the word "substantially" as a qualifier to certain features or effects in this disclosure is intended to simply mean that any deviations are within tolerances that would normally be expected by the skilled person in the relevant field.

The use of the word "generally" as a qualifier to certain features or effects in this disclosure is intended to simply mean - for a structural feature: that a majority or major portion of such feature exhibits the characteristic in question, and - for a functional feature or an effect: that a majority of outcomes involving the characteristic provide the effect, but that exceptionally outcomes do no provide the effect.

The present disclosure provides an electronic system for detecting presence of liquid in an appliance interface, a method for detecting presence of liquid in an appliance interface, an appliance interface, and a medical device comprising the electronic system.

In a first aspect of the invention, an electronic system for detecting presence of liquid in an appliance interface is disclosed. The electronic system comprises an appliance interface and an electronic device couplable to the appliance interface. The appliance interface comprises at least one primary appliance terminal, a reference appliance terminal, and a dielectric region separating the at least one primary appliance terminal and the reference appliance terminal. The electronic device comprises at least one primary device terminal configured to connect to the at least one primary appliance terminal, and a reference device terminal configured to connect to the reference appliance terminal. The electronic device is configured to generate and apply an electrical reference signal to the reference appliance terminal and to detect an electrical primary signal from the at least one primary appliance terminal. Further, the electronic device is configured to compare the primary signal with the reference signal, and, in accordance with at least a component of the primary signal being identical to the reference signal, determine that liquid is present in the appliance interface.

In the following, the electrical reference signal and the electrical primary signal is referred to as the reference signal and the primary signal, respectively. By an electrical signal is meant a signal conveying information composed of a (varying) electrical parameter over time, such as voltage (V), and/or current (I) as a function of time (t).

By liquid is meant any liquid substance, in particular any electrically conducting liquid substance, since such liquid is particularly relevant in settings involving electricity. For example, by liquid is meant water, such as tap water, sea water, body fluids, and liquids encountered on a daily basis, such as beverages. In embodiments, the electronic system is configured to detect presence of a conducting medium in the appliance interface, whereby is meant any substance, fluid or solid, present in the appliance interface.

An appliance interface is used in a variety of different settings, such as in electrical connectors comprising a plug (male component) and a socket (female component). Thus, by an appliance interface is meant the point at which one or more conductors (e.g., electrodes) of an appliance, such as a component, device, or network, comes to an end. Thus, the appliance interface facilitates an electrical (galvanic) connection between an appliance and another component, e.g. the electronic device according to the first aspect of the invention. The appliance interface comprises at least one primary appliance terminal and a reference appliance terminal. For example, the appliance interface comprises at least two, such as two, primary appliance terminals, and a reference appliance terminal.

In embodiments, by an appliance is meant a component, device, or network. In embodiments, the appliance interface is an appliance interface of a sensor assembly comprising one or more electrodes, such as two electrodes for each sensor provided in the sensor assembly. Thus, in embodiments, the appliance is a sensor assembly. In embodiments, the sensors are configured to detect changes in resistance/conductance and/or capacitance, such as when a voltage is applied across two (distinguishable) primary appliance terminals of the appliance interface connected to two electrodes forming a sensor (e.g., one of the two electrodes is grounded relative to the other).

In embodiments, the appliance interface comprises/forms part of a plug for receiving/entering a socket. In embodiments, the appliance interface comprises/forms part of a socket for receiving a plug. In embodiments, the appliance interface comprises/forms part of a port for enabling electrical communication between an electronic device and an appliance, such as a component, device, or network, connected to the appliance interface. In an embodiment, the appliance interface is substantially planar. Thus, terminals of the appliance interface may be exposed contact pads/points on a planar surface, the contact pads being adapted for receiving corresponding terminals of a corresponding connector, e.g. the electronic device. Providing a planar appliance interface may be particularly useful for use/incorporation in substantially planar appliances, such as medical devices for attachment to the skin, such as ostomy appliances or wound dressings. In embodiments, the appliance interface (male) is adapted to be inserted into a slit (device interface) formed in the electronic device (female). In embodiments, a clamping mechanism of the electronic device releasably secures the appliance interface in the device interface. In embodiments, the (planar) appliance interface has a thickness less than 5 mm, such as 5 mm, 4 mm, or 3 mm, or less than 2 mm, such as 1 mm or 0.5 mm. In embodiments, the appliance interface extends less than 25 mm in a first direction, and less than 25 mm in a second direction being perpendicular to the first direction. Thereby is provided an appliance interface with dimensions <25 mm x <25 mm in a two-dimensional plane, and a thickness <5 mm.

In embodiments, by liquid present in the appliance interface is meant that liquid is present on a surface of the appliance interface, or in the interface formed between the appliance interface and the electronic device, when the latter is coupled to said appliance interface.

Electronic devices are used in a variety of different settings, such as in electrical connectors comprising a plug (male) and a socket (female). In particular, within the present context, the electronic device comprises a device interface comprising the corresponding gender to the appliance interface, such that the electronic device (in particular the device interface) is couplable to the appliance interface. For example, in embodiments, the appliance interface comprises the male part, and the electronic device (device interface) comprises the female part, or the appliance interface comprises the female part and the electronic device comprises the male part. In a preferred embodiment, the appliance interface is considered a plug (male part) for the device interface of the electronic device (socket, female part). An electronic device within the present context can be a device capable of controlling the appliance comprising the appliance interface, e.g. through a processor and a power unit embodied in the electronic device. In embodiments, by an electronic device within the present context is meant a wireless/cordless personal device, i.e. a device easy to handle and having dimensions making it suitable for attachment to appliances used in everyday life, including medical devices, such as ostomy appliances and wound dressings. In embodiments, the electronic device is a monitor device for receiving sensory inputs, such as from sensors connected to the appliance interface. In embodiments, a maximum dimension, such as a length, of the electronic device is 10 cm, or 8 cm, or 6 cm. In embodiments, the electronic device is crescent-shaped.

jointly, the appliance interface and the electronic device is denoted the connectors, where it is envisioned that the appliance interface comprises one part of the connectors, and the device interface of the electronic device comprises the corresponding second part of the connectors. In the following, the electronic device is said to be couplable to the appliance interface, whereby it is meant that a device interface of the electronic device is couplable to the appliance interface. In particular, by couplable is meant that a galvanic electrical connection may be formed between the electronic device and the appliance interface.

In embodiments, the appliance and the appliance interface thereof are considered a passive part, meaning that power, such as voltage, needed to control the appliance is provided by means of the electronic device, which can therefore be considered an active part. Details on the electronic device is disclosed below.

Presence of liquid in an appliance interface, in particular when coupled to a corresponding connector (e.g., electronic device) and a voltage is applied, can cause malfunctioning of the connection and/or appliance, and/or risk of injury to the operator/user. Further, the presence of liquid in the interface can cause interference, false signals, or noise, such that signals pertaining to the appliance are unreliable. Thus, a need exists to provide detection and determination of the presence of liquid in the appliance interface when coupled to a corresponding socket/plug, such as an electronic device. Such ability is particularly desirable in medical devices, such as ostomy appliances, wound dressings, and urinary catheters, where exposure to body fluids and/or water (personal hygiene) is a likely outcome of usage. Based on a detection of liquid, remedying actions can be applied, such as by requesting the user to clean the interface, entering a safe mode of the electronic device, or disregarding some or all signals pertaining to the appliance, since such signals can be faulty.

The electronic device can be coupled to the appliance interface in a variety of ways, including by means and methods known in the art, such as suitable mechanical couplings, such as press fit or snap fit. By coupled is meant that the electronic device can be coupled to the appliance interface in such a way that an electrical (galvanic) connection between the device and the appliance interface is established. In embodiments, the electronic device is releasably couplable to the appliance interface, such that the electronic device can be coupled to the appliance interface and subsequently be decoupled/detached from the appliance interface. In embodiments, the electronic device and/or appliance interface are provided with means for securing/fastening the connectors together in a coupled configuration, and such that the electronic device and the appliance interface can be decoupled by actuating a force on a coupling mechanism.

The appliance interface comprises at least one primary appliance terminal. The at least one primary appliance terminal is electrically conductive. By a primary appliance terminal is meant a terminal constituting the endpoint of an electrode of the appliance comprising the appliance interface. For example, where the appliance interface is an appliance interface of an electrode of a sensor, the primary appliance terminal constitutes the terminal allowing to control the electrode, e.g. by means of an electronic device couplable to the appliance interface. The appliance interface comprises at least one primary appliance terminal, such as one, two, three, four, five, six, seven, nine, or ten primary appliance terminals. For example, where the appliance comprising the appliance interface comprises N separate parts, such as N electrodes, the appliance interface can be provided with N primary appliance terminals, thereby providing means for communicating separately with each of the N separate parts, such as N electrodes.

In embodiments, the appliance interface comprises at least two primary appliance terminals. For example, by providing at least two primary appliance terminals, one of the at least two primary appliance terminals can be grounded - by means of the electronic device - relative to the remaining primary appliance terminal(s). In embodiments, one of the at least two primary appliance terminals is a common ground for the remaining primary appliance terminal(s).

In embodiments, the at least one primary appliance terminal is shaped as a planar contact pad, such that an electrical galvanic connection between an electronic device and the appliance comprising the appliance interface can be established by bringing a corresponding terminal (contact pad thereof) of the electronic device into physical (galvanic) contact with the contact pad. In embodiments, the contact pad is circular or rectangular. In embodiments, a maximum dimension of the contact pad is 10 mm, or 8 mm, or 6 mm, or 5 mm, or 4 mm, or 3 mm, or 2 mm, or 1 mm. By providing the at least one primary appliance terminal as a planar contact pad, the appliance interface as such may be provided as substantially planar. In embodiments, the at least one primary appliance terminal is shaped as a pin configured to engage with a corresponding socket of the device interface. In embodiments, two or more primary appliance terminals are arranged adjacent each other but electrically insulated from each other. For example, two or more primary appliance terminals are separated (by a dielectric material), such as in a plane, by a distance of at least 0.5 mm, such as 0.5 mm, or at least 1 mm, or at least 2 mm, or at least 3 mm.

The electronic device is configured to detect an electrical primary signal through the at least one primary appliance terminal. In embodiments, the primary signal is a composite signal comprising two or more component signals (in short denoted components). For example, two or more components of the primary signal can arise from the appliance, such as in a multiplexer configuration. The primary signal can comprise a component signal from the appliance (an appliance signal) and a component signal pertaining to the reference signal, such as a component being identical to the reference signal, and hence indicate presence of liquid (to be described in detail below). Thus, in embodiments, appliance signal(s) and the reference signal are superimposed. In the following, a reference to appliance signal (singular) can likewise be a reference to appliance signals (plural) and vice versa, unless otherwise specified or understood from the context.

In embodiments, the at least one primary appliance terminal is configured to transmit/relay/output electrical signals to/from the appliance comprising the appliance interface. In the following, signals arising/pertaining to the appliance comprising the appliance interface are denoted appliance signal(s). Thus, the primary appliance terminal is configured to output/input a composite signal, the composite signal comprising appliance signals (one or more components) and, in accordance with the presence of liquid in the appliance interface, a component pertaining to the reference signal. If the component pertaining to the reference signal (i.e., the component being identical to, or comprises a characteristic of, the reference signal) is present in the (composite) primary signal, it is indicative of a presence of liquid. This is described further in the following. If liquid is not present in the appliance interface, the primary signal can either be absent (if no appliance signals are generated by the appliance), or the primary signal can comprise appliance signal(s) only. In other words, the reference signal is detectable in the primary signal.

The appliance interface comprises a reference appliance terminal. The reference appliance terminal is electrically conductive. In embodiments, the reference appliance terminal does not constitute an endpoint of an appliance comprising the appliance interface. Rather, the reference appliance terminal is provided in/on the appliance interface but is not configured to establish an electrical connection between the electronic device coupled to the appliance interface and an electrode of the appliance comprising the appliance interface. Thus, in embodiments, the reference appliance terminal is electrically insulated from any other conductive parts (in particular the at least one primary appliance terminal) of the appliance interface and parts of the appliance connected thereto. In embodiments, the reference appliance terminal is shaped as a planar contact pad, such as shaped identically to the at least one primary appliance terminal. In embodiments, the reference appliance terminal is shaped as a pin. The reference appliance terminal is configured to receive a reference signal from, and generated by, the electronic device when the latter is coupled to the appliance interface. In embodiments, multiple, such as at least two, reference appliance terminals are provided in the appliance interface, such as to provide a back-up reference appliance terminal in case of failure of a first reference appliance terminal, or to provide a control.

A dielectric region separates the at least one primary appliance terminal and the reference appliance terminal, such as a planar dielectric region where the appliance interface is planar and comprises planar contact pads. Thereby, at least in a dry state of the appliance interface, electrical signals (e.g., current) cannot be transmitted (e.g., flow) to/from the reference appliance terminal from/to the at least one primary appliance terminal. In embodiments, a dielectric region encloses each of the at least one primary appliance terminals. By enclose is meant to surround, such as in the plane, each of the at least one primary appliance terminals, e.g. when the appliance interface is substantially planar, and when seen in a direction normal to the plane of the appliance interface. Thus, by enclose is not meant to encapsulate and thereby fully insulate the at least one primary appliance terminal from the entire surroundings.

In embodiments, the dielectric region serves to insulate the at least one primary appliance terminal from another (if provided) and/or to insulate the at least one primary appliance terminal from the reference appliance terminal. For example, where the at least one primary appliance terminal is a planar contact pad, the dielectric region can be provided along the periphery of such contact pad, such as within 1 mm of the periphery of the primary appliance terminal. In embodiments, multiple dielectric regions are provided, such as when the (planar) appliance interface comprises two or more primary appliance terminals, a (planar) dielectric region can surround/enclose each of said two or more (planar) primary appliance terminals. Thus, in embodiments, a dielectric region exists for each of the primary appliance terminals.

In embodiments, the dielectric region comprises a dielectric material, such as a dielectric (co)polymer material, such as a (co)polymer material not capable of absorbing liquids, such as isobornyl acrylate. In embodiments, the dielectric region comprises oxides or nitrides, such as silicon dioxide and silicon nitride, or similar dielectric materials known and used for their dielectric properties in planar (silicon) processing. In embodiments, the dielectric region comprises a dielectric ceramic material. In embodiments, the dielectric material is printable, such as to provide fast and easy manufacturing. In embodiments, the dielectric material forms a substrate for the terminals, such that the terminals are provided, e.g., printed, on a surface of the dielectric material. In embodiments the dielectric material is stretchable, flexible, bendable, and/or resilient, such as to provide a flexible appliance interface. In embodiments, the stretchability is provided by the dielectric material being an adhesive composition.

The electronic device comprises at least one primary device terminal and a reference device terminal. The at least one primary device terminal and the reference device terminal are electrically conductive. The at least one primary device terminal is configured to connect to the at least one primary appliance terminal, and the reference device terminal is configured to connect to the reference appliance terminal. By connect is meant to establish a (physical, galvanic) electrical connection between the respective terminals such as to facilitate transmission of electrical signals. In embodiments, the device terminals (primary and reference terminals) connect/mate with the appliance terminals (primary and reference terminals) in a one-to-one manner, such that the electronic device comprises the same number of terminals as the appliance interface. In embodiments, the appliance interface and the electronic device comprises a different total number of terminals, but at least a primary device terminal configured to connect to a primary appliance terminal, and a reference device terminal configured to connect to a reference appliance terminal. The at least one primary device terminal and the reference device terminal are electrically insulated from each other, e.g. by a device dielectric region.

In embodiments, the at least one primary device terminal and the reference device terminal are equal or corresponding in shape to the at least one primary appliance terminal and the reference appliance terminal. In embodiments, the device terminals are resilient or supported by a spring to force the terminals into contact with the appliance terminals.

In embodiments, the at least one primary device terminal is connected to a first circuitry of the electronic device, and the reference device terminal is connected to a second circuity of the electronic device. In embodiments, the first circuity is configured to apply a voltage across two or more primary terminals or to gather electrical primary signals from the at least one primary appliance terminal. In embodiments, the second circuitry is configured to generate a reference signal to be conducted to the reference appliance terminal. In embodiments, the circuitries are controlled by a processor.

In embodiments, the terminals, including the at least one primary appliance terminal, the reference appliance terminal, the at least one primary device terminal, and the reference device terminal comprise one or more of metallic (e.g.

silver, copper, gold, titanium, aluminium, stainless steel), ceramic (e.g. ITO), polymeric (e.g. PEDOT, PANI, PPy), and carbonaceous (e.g. carbon black, carbon nanotube, carbon fibre, graphene, graphite) materials. In embodiments, the at least one primary appliance terminal and the reference appliance terminal are made of a printable material, such as silver and/or a carbonaceous material, such as carbon. In embodiments, the at least one primary appliance terminal and the reference appliance terminal are flexible, bendable and/or stretchable, such as to provide a stretchable appliance interface, e.g. when printed onto a flexible/bendable/stretchable dielectric material forming a substrate for said terminals.

In embodiments, the electronic device comprises a housing, a processor, a memory, a power unit, and a circuitry. The power unit can be configured to apply a voltage across two or more terminals, such as to ground one of the two or more terminals relative to the remaining terminal(s). In embodiments, the power unit comprises a battery. In embodiments, the power unit comprises a charging circuity connected to the battery and terminals of a charging interface for charging the battery via the charging interface. In embodiments, the charging interface is the device interface, such that the battery can be charged by coupling the electronic device to a charger unit. In embodiments, two or more terminals of the device interface are configured to charge the battery.

In embodiments, the electronic device comprises a sensor unit with one or more sensors. The sensor unit is connected to the processor for feeding sensor data to the processor. In embodiments, the sensor unit comprises an accelerometer for sensing acceleration and provision of acceleration data to the processor. In embodiments, the sensor unit comprises a temperature sensor for provision of temperature data to the processor.

In embodiments, the electronic device comprises a transmission interface. In embodiments, the transmission interface is configured as an accessory interface for connecting, e.g. wirelessly connecting, the electronic device to one or more accessory devices. In embodiments, the transmission interface comprises an antenna and a wireless transceiver, e.g. configured for wireless communication at frequencies in the range from 2.4 to 2.5 GHz. In embodiments, the wireless transceiver is a Bluetooth transceiver, i.e. the wireless transceiver can be configured for wireless communication according to a Bluetooth protocol, e.g. Bluetooth Low Energy, Bluetooth 4.0, and/or Bluetooth 5. An accessory device can be a mobile phone, e.g. smartphone, or other handheld device, or a wearable, such as a watch or other wrist-worn electronic device. Thereby, the electronic device of the present disclosure can be configured to communicate with an accessory device, such as to allow such accessory device to visualize data gathered by the electronic device, e.g. when the latter is coupled to an appliance comprising an appliance interface as disclosed. In embodiments, the transmission interface comprises a loudspeaker and/or a haptic feedback element for provision/transmission of an audio signal and/or haptic feedback to a user, respectively.

The electronic device is configured to generate and apply a reference signal to the reference appliance terminal through the reference device terminal. In embodiments, by generating and applying a signal to the reference appliance terminal is meant that the electronic device is configured to generate such signal and to conduct the signal to the reference device terminal. When the electronic device is coupled to the appliance interface, an electrical connection between the reference device terminal and the reference appliance terminal is established, whereby the signal is conducted to the reference appliance terminal. Thus, in embodiments, by being configured to is meant that the electronic device can couple to the appliance interface in such a way that an electrical connection between the reference device terminal and the reference appliance terminal is established. In embodiments, the signal is generated by means of a driver or the processor and the power unit, such that the power unit provides the sufficient current or voltage, and the driver or processor generates an appropriate reference signal. In preferred embodiments, the reference signal has a certain characteristic, such as a certain electrical waveform or a certain frequency, making it a distinguishable component signal of a possible composite primary signal.

The electronic device is configured to detect a primary signal from the at least one primary appliance terminal through the at least one primary device terminal. In embodiments, by detecting a signal from the at least one primary appliance terminal is meant that the electronic device is configured to detect such a signal, e.g. by detecting changes in current or voltage over time indicative of such signal. When the electronic device is coupled to the appliance interface, an electrical connection between the primary appliance terminal and the primary device terminal is established, whereby a signal can be conducted from said primary appliance terminal to said primary device terminal. Thus, in embodiments, by being configured to is meant that the electronic device can couple to the appliance interface in such a way that an electrical connection between the primary appliance terminal and the primary device terminal is established.

In embodiments, the primary signal is a composite signal comprising a component (appliance) signal from the appliance and a component signal from the reference appliance terminal (such component signal being the reference signal if liquid in the appliance interface provides a liquid path/bridge for conducting the reference signal from the reference appliance terminal to the primary appliance terminal). In embodiments, such as in a dry state of the appliance interface, the primary signal may comprise component signal(s) from the appliance (appliance signal(s)) only.

As previously described, the electronic device is configured to compare the primary signal, or components thereof, with the reference signal. Thus, electronic device is configured to compare the two signals and determine their resemblance. In embodiments, the electronic device is configured to identify a component signal of the primary signal, and to compare such component signal with the reference signal. If at least a component of the primary signal is identical to the reference signal (e.g., if at least a component of the primary signal shares a certain characteristic with the reference signal), it is indicative of liquid in the appliance interface.

Alternatively, in embodiments, the electronic device is configured to compare the primary signal, or components thereof, with the reference signal, and, in accordance with at least a component of the primary signal comprising a characteristic pertaining to the reference signal, determine that liquid is present in the appliance interface. For example, the characteristic can be a certain feature of the reference signal not expected to be generated by the appliance. Thus, the presence of the characteristic pertaining to the reference signal is indicative of liquid in the appliance interface.

According to embodiments, only through the presence of (electrically conducting) liquid in the appliance interface can the primary signal, or a component thereof, be identical to the reference signal or comprise a characteristic pertaining to the reference signal. In other words, since the reference appliance terminal is (structurally and electrically) separated/insulated from the primary appliance terminal by a dielectric region, a resemblance between the reference signal and the primary signal (or component thereof) indicates a presence of liquid: the liquid bridges from the reference appliance terminal to the primary appliance terminal and thereby facilitates the conduction of the reference signal from the reference appliance terminal to the primary appliance terminal, whereby the primary signal (or a component thereof) is identical to, or comprises a characteristic of, the reference signal. In embodiments, resemblance is determined within a certain time period, such as by comparing the past (trailing) 1 second (or other, such as between 0.1 second and 30 seconds, such as 10 seconds) of the primary signal with the past (trailing) 1 second (or other, such as between 0.1 second and 30 seconds, such as 10 seconds) of the reference signal.

In embodiments, by identical is meant that the signals share a strong resemblance, such as at least 80 %, or at least 90 %, of the measuring points are coinciding. In embodiments, by identical is meant that a characteristic, such as a waveform, of, or contained in, the primary signal (or a component thereof) resembles the characteristic, such as the waveform, of the reference signal, in a qualitative and/or quantitative manner. By a qualitative manner is meant that a person skilled in the art (and/or an artificial intelligence (AI) sufficiently trained in the specific use) is capable of identifying and/or distinguishing the characteristic, such as the waveform, from noise and/or appliance signals in a composite signal. Thus, the reference signal (i.e., the characteristics, such as the waveform, thereof) is purposively (pre-)selected to be distinguishable, e.g. by software of the electronic device and/or by a skilled person, from appliance signals contained in the (composite) primary signal. For example, the reference signal can be selected by the software of the electronic device, such as through machine learning considering the appliance signals and, over time, generates a reference signal easily distinguishable from the appliance signals contained in the composite primary signal. Alternatively, the reference signal is selected by the manufacturer of the electronic system, e.g. through the manufacturer's knowledge of the expected appliance signals.

Thereby is provided an electronic system for detecting presence of liquid in an appliance interface of the electronic system - a water/liquid ingress detection system. The disclosed electronic system is a plug-and-socket type of system, where the means for detecting such presence of liquid is shared between a (passive) appliance interface and an (active) electronic device couplable thereto.

In alternative embodiments, the electronic device is configured to apply a reference signal to the primary appliance terminal and to detect a primary signal from the reference appliance terminal, i.e., the above-outlined process in reverse. Thus, in embodiments, the process outlined above works in reverse, and the same principles and conclusions apply.

In an embodiment, the reference signal has a first electrical waveform.

By an electrical waveform (also referred to as waveform only), is meant that the voltage, current, or electromagnetic field associated with the (electrical) signal varies (e.g., periodically) in time. The waveform can be either periodic or irregular. In embodiments, the reference signal is an alternating electrical signal.

In embodiments, the waveform is generated by means of a driver or the processor controlling the power unit, such that the power unit provides the sufficient variances in voltage, current, or electromagnetic field representing the selected waveform.

In embodiments, the first electrical waveform has a unique or unusual waveform unlikely to be caused/generated by the appliance, i.e., unlikely to be contained in the appliance (component) signal of the primary signal. Thus, in embodiments, the waveform constitutes a characteristic of the reference signal. Thereby is provided means for distinguishing the reference signal from appliance signal(s) in the (composite) primary signal. In other words, in embodiments, the electronic device is configured to look for the specific (unique/unusual) first electrical waveform in the primary signal potentially also comprising appliance signals. Thus, appliance signals can be detected and analysed and a presence of liquid in the appliance interface can be detected simultaneously (i.e., the electronic device searches for the reference signal in the primary signal while detecting and analysing appliance signals). In alternative embodiments, no appliance signals are generated while searching for the reference signal in the primary signal. As was also discussed above, a presence of liquid can be determined upon detection of (at least a component of) the primary signal being identical to the reference signal. In embodiments, the electronic device is configured to control (e.g. to apply a voltage or current and detect changes thereof/detect appliance signals) the appliance while simultaneously applying the electronic system as disclosed, i.e. detecting presence of liquid in the appliance interface. Whereas appliance signals can comprise noise and/or certain characteristics pertaining to the appliance, the first electrical waveform of the reference signal can be selected such that it is distinguishable (in particular its characteristics, such as its waveform) from expected appliance signals arising from the appliance. Thereby is provided means for simultaneously controlling the electronic system for detecting presence of liquid in the appliance interface and controlling the appliance.

In embodiments of the appliance interface comprising two or more primary appliance terminals, the electronic device is configured to either simultaneously or consecutively search the primary signal for the component signal pertaining to the reference signal. Thus, in embodiments, the electronic device is configured to detect and analyse primary signals pertaining to two or more primary appliance terminals simultaneously. In embodiments, the electronic device is configured to determine which primary appliance terminal is exposed to liquid in the appliance interface, and, optionally, disregard the specific primary signal pertaining to this primary appliance terminal and thus the connected electrode of the appliance.

In an embodiment, the first electrical waveform is selected from a sine waveform, a square waveform, a rectangular waveform, a triangular waveform, and a saw-toothed waveform.

In embodiments, the waveform of the reference signal is selected based on the expected appliance signals arising from the appliance comprising the appliance interface, such that the selected waveform is a waveform not expected to be found in the appliance signals. Thereby, the selected waveform can be distinguished from the appliance signals, for the reasons and benefits discussed above.

In embodiments, the electronic device can adapt the waveform of the reference signal over time, e.g. as a result of a machine learning algorithm considering the appliance signals and designing a waveform, or selecting a pre-stored waveform, distinguishable from such appliance signals, e.g. such that it is at least distinguishable to the software of the electronic device. In embodiments, the waveform is selected by the manufacturer of the electronic system and/or the appliance. For example, the manufacturer can select a waveform generally known from experience and/or testing not to be found in the appliance signals.

In embodiments, periodic waveforms are preferred, such as the waveforms outlined below.

A sine waveform $f(t, \lambda, a, \phi)$ can be represented as

$$f(t, \lambda, a, \phi) = a \sin \frac{2\pi t - \phi}{\lambda}$$

A square (and rectangular, with modifications to duty) waveform $g(t, \lambda, a, \phi)$ can be represented as

$$g(t, \lambda, a, \phi) = \begin{cases} a, & (t - \phi) \bmod \lambda < \text{ duty} \\ -a, & \text{otherwise} \end{cases}$$

A triangular waveform $h(t, \lambda, a, \phi)$ can be represented as

$$h(t, \lambda, a, \phi) = \frac{2a}{\pi} \arcsin \sin \frac{2\pi t - \phi}{\lambda}$$

A saw-toothed waveform $k(t, \lambda, a, \phi)$ can be represented as

$$k(t, \lambda, a, \phi) = \frac{2a}{\pi} \arctan \tan \frac{2\pi t - \phi}{2\lambda}$$

In the above equations, $t$ is time, $\lambda$ is wavelength, a is amplitude and $\phi$ is phase. The wavelength (frequency), amplitude and phase can be modified/selected to further make the reference signal distinguishable from the appliance signals.

In an embodiment, the reference appliance terminal comprises a contact pad and an enclosing portion. The enclosing portion at least partly encloses the at least one primary appliance terminal.

The contact pad and the enclosing portion are both electrically conductive and connected, such that electrical signals can be conducted/transmitted from the contact pad and into the enclosing portion, and vice versa.

In embodiments, the contact pad is planar. In embodiments, the appliance interface is planar, such that the contact pad may flush with other contact pads, such as contact pads of one or more primary appliance terminals. In embodiments, the enclosing portion is planar, such that the enclosing portion at least partly encloses the at least one primary appliance terminal in the plane of the appliance interface.

In embodiments, by enclosing is meant to surround the at least one primary appliance terminal, e.g. in embodiments where the appliance interface is substantially planar, such that when seen in a direction normal to the plane of the appliance interface (i.e., a top view, looking down at the appliance interface), the enclosing portion at least partly surrounds/encloses the at least one primary appliance terminal (a dielectric region still separates the at least one primary appliance terminal from the enclosing portion). Thus, for illustrative purposes, the enclosing portion may resemble an arm/leg or a tentacle for increasing the reach of the contact pad of the reference appliance terminal.

In embodiments, the contact pad of the reference appliance terminal is shaped and dimensioned to resemble the at least one primary appliance terminal, such as a contact pad of the at least one primary appliance terminal.

In embodiments, the enclosing portion comprises one or more legs extending from the contact pad and at least partly encloses the at least one primary appliance terminal, such as in the plane. For example, in embodiments where the appliance interface comprises a substantially planar surface with the appliance terminals arranged thereupon, the legs can be arranged along, e.g. parallel to, at least one, or at least two edges of the at least one primary appliance terminal. Thereby, a liquid can be detected according to the first aspect of the invention if it forms a bridge across the dielectric region from a leg of the reference appliance terminal to the at least one primary appliance terminal. Thereby, the reference signal can be conducted from the electronic device through the contact pad, into the leg, through the liquid, and eventually be contained in the primary signal. Thus, the enclosing portion increases the ability of the electronic system to detect a presence of liquid, since liquid can form a bridge from any point of a leg connected to the contact pad of the reference appliance terminal to the primary appliance terminal.

In embodiments, the enclosing portion is a coherent region/surface of the appliance interface. In corresponding embodiments, the dielectric region surrounds the (immediate vicinity of) each of the at least one primary appliance terminals, such that the remaining surface of the appliance interface (in embodiments of this being planar as discussed previously) is covered by the enclosing portion of the reference appliance terminal. In other words, in embodiments, a dielectric region encloses each of the at least one primary appliance terminals.

A designated part of the enclosing portion can be considered to be the contact pad where the reference device terminal is configured to touch/form an electrical connection to the reference appliance terminal. For example, the contact pad can be considered a reinforced region of the reference appliance terminal, which otherwise comprises the enclosing portion. Thereby, a conducting liquid merely needs to bridge across the dielectric region surrounding the immediate vicinity of the at least one primary appliance terminal. In embodiments, by immediate vicinity is meant that the dielectric region extends less than 3 mm away from a periphery of the at least one primary appliance terminal, such as less than 1 mm. Thus, by providing the enclosing portion as a coherent region/surface covering a substantial portion of the appliance interface (e.g., more than 25 % or more than 50 % of the surface area of the appliance interface in embodiments of the appliance interface being planar), presence of liquid is increasingly likely to be detected.

In an embodiment, the reference device terminal is configured to connect to the contact pad of the reference appliance terminal.

In embodiments, the contact pad is a designated area of the enclosing portion of the reference appliance terminal, such as a reinforced designated area. In embodiments, by reinforced is meant that the contact pad is made of a different conductive material than the enclosing portion, such as a more durable conductive material than the enclosing portion, or the contact pad may be provided with a thicker layer of conductive material, such that the contact pad is configured to be in physical (galvanic) contact with the reference device terminal without causing degradation of the contact pad.

In an embodiment, the at least one primary appliance terminal is a terminal of an electrode of a sensor. Thus, in embodiments, the appliance comprising the appliance interface and hence the at least one primary appliance terminal is a sensor, such as a sensor assembly comprising at least one sensor.

In embodiments, the primary appliance terminal is the endpoint of an electrode of a sensor. In embodiments, the appliance interface comprises a plurality of primary appliance terminals, such as two or more primary appliance terminals, forming the endpoint of a plurality of electrodes, such as two or more electrodes, of, or for, a sensor. In embodiments, the at least one primary appliance terminal is a terminal of an electrode of a sensor of a sensor assembly. In embodiments, a sensor comprises two electrodes. In embodiments, the electronic device is configured to apply a voltage across the two electrodes of a sensor (e.g., to ground one of the two electrodes relative to the other) and to monitor changes in relevant electrical parameters, such as resistance or capacitance, in order to sense changes in the environment/surroundings of the sensor (sensor assembly).

In embodiments, the appliance, such as the sensor assembly, is substantially planar. In embodiments, the sensor assembly and the appliance interface are arranged in the same geometrical plane. In embodiments, the sensor assembly is stretchable/flexible. In embodiments, the electrode(s) of the sensor assembly is arranged, e.g. printed, on a stretchable substrate, such as a polymer material. In embodiments, the appliance interface and the electrode(s) of the sensor assembly are printed in a single process, e.g. printed on a support layer/film. In an embodiment, the appliance interface is arranged on a stretchable substrate. By a stretchable substrate is meant a flexible and/or elastic substrate, such as a polymer material or an adhesive material for a medical device, such as a base plate for an ostomy appliance, or a wound dressing. In embodiments, the stretchable substrate comprises the dielectric region of the appliance interface.

In embodiments, the adhesive material, e.g. an adhesive layer, is made of a first composition. In embodiments, the first composition comprises one or more polyisobutenes and/or styrene-isoprene-styrene. In embodiments, the first composition comprises one or more hydrocolloids. In embodiments, the first composition comprises one or more water soluble or water swellable hydrocolloids. In embodiments, the first composition is a pressure sensitive adhesive composition suitable for medical purposes comprising a rubbery elastomeric base and one or more water soluble or water swellable hydrocolloids. In embodiments, the first composition comprises one or more polybutenes, one or more styrene copolymers, one or more hydrocolloids, or any combination thereof. The combination of the adhesive properties of the polybutenes and the absorbing properties of the hydrocolloids renders the first composition suitable for use in ostomy appliances. For example, the styrene copolymer can be a styrene-butadiene-styrene block copolymer or a styrene-isoprene-styrene block copolymer. Preferably, one or more styrene-isoprene-styrene (SIS) block type copolymers are employed. The amount of styrene block-copolymer can be from 5 % to 20 % of the total adhesive composition. The butene component is suitably a conjugated butadiene polymer selected from polybutadiene, polyisoprene. The polybutenes are preferably present in an amount of from 35 - 50 % of the total adhesive composition. Preferably, the polybutene is polyisobutylene (PIB). Suitable hydrocolloids for incorporation in the first composition are selected from naturally occurring hydrocolloids, semisynthetic hydrocolloids, and synthetic hydrocolloids. The first composition can comprise 20-60 % hydrocolloids. A preferred hydrocolloid is carboxymethyl cellulose (CMC). Optionally, the first composition can contain other components, such as fillers, tackifiers, plasticizers, and/or other additives.

In embodiments, the sensor (the sensor assembly) is embedded in a medical device, such that the sensor is provided as an integral part of the medical device. For example, the sensor can be fixed/incorporated within the material forming at least part of the medical device.

In embodiments, the medical device is an ostomy appliance. In embodiments, the ostomy appliance comprises a base plate comprising an adhesive layer for adhering the base plate to a peristomal skin surface of a user having an ostomy. In embodiments, the sensor assembly is configured to surround the ostomy and to detect moisture absorption in the first adhesive layer and/or to detect presence of liquid (such as output emanating from the ostomy) in the interface between the adhesive layer and the peristomal skin surface of the user. For example, the sensor assembly can be provided on a distal and/or proximal surface of the adhesive layer (by proximal is meant arranged closest to the skin surface, relative to another element (thus being distal), when worn by a user), e.g. sandwiched between a distal surface of the adhesive layer and a proximal surface of a polymeric top film protecting the adhesive layer.

In embodiments, the medical device is a wound dressing. In embodiments, the sensor assembly provides for detecting presence and/or propagation of exudate in the vicinity of a wound covered by the wound dressing. For example, the sensor assembly can be provided on a distal and/or proximal side of an absorbent core (by proximal is meant arranged between the absorbent core and the wound) of such wound dressing, e.g. sandwiched between a distal side of the absorbent core and a proximal side of a polymeric top film protecting the absorbent core.

In an embodiment, the appliance interface is disposable, and the electronic device is reusable. In embodiments, the appliance and the appliance interface thereof are disposable. For example, where the appliance is a sensor assembly embedded in a base plate of an ostomy appliance or in a wound dressing, it is desired to provide such base plate or wound dressing as a disposable item, e.g. a single-use item. By being couplable to the appliance interface, the electronic device can be decoupled and re-coupled to a new appliance interface of new appliance after disposal of the earlier appliance. Thereby, relatively expensive electronics can be provided in the electronic device, whereas the costs of the appliance interface and the associated appliance can be reduced to a minimum. Thereby is provided an electronic system comprising two parts, wherein one part is disposable and relatively cheap (the appliance interface, also referred to as a passive part), and wherein a second part is reusable and relatively expensive (the electronic device, also referred to as an active part), and wherein the electronic system retains the ability to detect a presence of liquid in the appliance interface. In embodiments, the appliance interface is planar, as previously discussed, in order to be provided in a planar appliance, such as a base plate or a wound dressing. Thereby, the electronic device and the appliance interface may be coupled in the plane of the appliance, thereby reducing the bulk size of the appliance when in use.

In an embodiment, the appliance interface comprises a first primary appliance terminal and a second primary appliance terminal, and the electronic device comprises a first primary device terminal and a second primary device terminal. In embodiments, the first primary device terminal is configured to establish an electrical connection to the first primary appliance terminal, and the second primary device terminal is configured to establish an electrical connection to the second primary appliance terminal. Thus, in addition to the appliance/device reference terminal, the appliance interface/electronic device comprise a first and a second primary appliance/device terminal.

In embodiments, the appliance interface comprises more than two primary appliance terminals, such as a third, a fourth, a fifth, a sixth, a seventh, an eight, a ninth, and/or a tenth primary appliance terminal. Correspondingly, in embodiments, the electronic device comprises more than two primary device terminals, such as a third, a fourth, a fifth, a sixth, a seventh, an eight, a ninth, and/or a tenth primary device terminal. In embodiments, the primary appliance terminals are configured to contact the primary device terminals in a one-to-one manner/configuration.

In an embodiment, the electronic system further comprises an appliance connected to the appliance interface. In embodiments, the appliance is a base plate of an ostomy appliance, a sensor patch for attachment to the proximal surface of a base plate, or a wound dressing, as previously discussed. According to the embodiment, the first primary appliance terminal is a terminal of a first electrode of the appliance, and the second primary appliance terminal is a terminal of a second electrode of the appliance. By connected to the appliance interface is meant that the appliance interface is an appliance interface of the appliance, such that the appliance and the appliance are electrically connected. In embodiments, the appliance is, or comprises, a sensor assembly. In embodiments, jointly, the first and the second electrode form a sensor of the sensor assembly. In embodiments, an electrode is a conductive wire or path, such that electricity or a voltage can be applied the first and the second electrode. In embodiments, electrodes are printable on a support layer, such as printable in a continuous process along with the terminals of the appliance interface.

According to the embodiment, the appliance interface comprises a first and a second primary appliance terminal, which, in combination with previous embodiments, are electrically connectable/couplable to corresponding first and second device terminals of the electronic device. Thereby, the electronic device can read and/or control (e.g., apply a voltage across) each of the first and second electrodes connected to the first and second appliance terminals, respectively, individually/independently. In other words, the primary appliance terminals are distinguishable to the electronic device.

In embodiments, the electronic device is a monitor device configured to monitor changes in one or more electrical quantities/parameters, such as voltage, resistance, resistivity, conductance, conductivity, and/or capacitance, pertaining to the electrodes of the appliance (e.g., a sensor). Such changes in electrical parameters can be attributed changes in the environment of the appliance (sensor), and thus provide valuable information to the electronic device, which can be analysed and communicated to a user of the device. For example, the electronic device may be configured to detect presence of stomal output on a proximal surface of a base plate, presence of moisture in the adhesive layer of a base plate, or the presence of exudate uptake in a foam material of a wound dressing.

In an embodiment, the electronic device is configured to apply a voltage across the first primary appliance terminal and the second primary appliance terminal. In embodiments, the first and the second primary appliance terminals are endpoints of a first and a second electrode, respectively, of the appliance, such as of a sensor assembly. Thus, by applying a voltage across the first and the second primary appliance terminal, a voltage is applied across the first and the second electrode. In embodiments, the two electrodes form a sensor. In embodiments, the electrodes are provided in a non-conductive medium or a liquid-absorbing medium, or exposed to the surroundings. In embodiments, the non-conductive medium or the liquid-absorbing medium is an adhesive composition, such as an adhesive layer of a medical device, such as a base plate of an ostomy appliance.

By applying a voltage across the electrodes, it is possible to monitor changes in the resistance in the non-conductive medium or the liquid-absorbing medium or the surroundings, the resistance being indicative of physical or chemical changes in the medium or the surroundings. Thereby is provided a sensor (sensor assembly) having an appliance interface as described, and the sensor being configured to detect changes in resistance indicative of changes in the surroundings, and the appliance interface being configured to detect presence of liquid according to the first aspect of the invention.

In an embodiment, the electronic device is configured to detect a short-circuit across the first primary appliance terminal and the second primary appliance terminal. When applying a voltage across the terminals, and hence electrodes thereof, it is possible that liquid bridging across the electrodes causes a short-circuit. Such presence of liquid can be indicative of changes in the surroundings of the electrodes (sensor) of the appliance, and hence be valuable information to the user, e.g. where such presence of liquid is unwanted, e.g. in the interface between a peristomal skin surface of a user and a first adhesive layer of a base plate of an ostomy appliance.

In an embodiment, the electronic device is configured to, in accordance with a detection of a short-circuit across the first primary appliance terminal and the second primary appliance terminal, determine that liquid is present in/near the appliance. In embodiments, the appliance is a sensor assembly as described above.

The occurrence/detection of a short-circuit across two primary appliance terminals can be caused by two different events: either due to liquid bridging across electrodes in the appliance (e.g. a sensor of the sensor assembly) connected to the two primary appliance terminals (which can be a desired functionality of the monitor aspect of the electronic device), or due to liquid in the appliance interface bridging from one primary appliance terminal to another (which is unwanted due to reasons previously disclosed and an object of the first aspect of the invention). However, due to the electronic device being capable of applying a reference signal to the reference appliance terminal, compare a detected primary signal with the reference signal and determining if liquid is present in the appliance interface based on the resemblance between the signals, it is possible to distinguish a short-circuit across the electrodes of the appliance from a short-circuit due to liquid in the appliance interface. In other words, presence of liquid in the appliance interface is detected by means different from detecting a short-circuit, meaning that the detection of a short-circuit can be attributed to the appliance comprising the appliance interface rather than the primary appliance terminals of the appliance interface as such. In further other words, the electronic system according to the embodiment is configured to differentiate a short-circuit in the appliance comprising the appliance interface from a short-circuit in the appliance interface as such (e.g. due to the presence of liquid in said appliance interface). The ability to differentiate such short-circuit relies on the application, detection, and comparison of electrical signals having a certain characteristic, such as a certain waveform. It is appreciated that whereas a short-circuit is discussed, the present disclosure may also facilitate the detection of minor changes in electrical characteristics of the electrodes of the appliance, and that such minor changes may likewise be differentiated from the signal being indicative of liquid in the appliance interface as such.

In an embodiment, the electronic system further comprises an accessory device. According to the embodiment, the electronic device is configured to, in accordance with determining that liquid is present in the appliance interface, transmit a device signal to the accessory device.

In an embodiment, the accessory device is configured to, in accordance with receiving the device signal from the electronic device, visualize the presence of liquid in a user interface of the accessory device, such as a graphical user interface.

In embodiments, the device signal is a wireless signal transmitted by means of a transmission interface of the electronic device, as previously disclosed.

As previously discussed, presence of liquid in the appliance interface can constitute a risk for the user and/or the equipment (e.g., the electronic device) or can cause faulty/unreliable (appliance) signals. Thus, it is desired to provide means for communicating such presence of liquid to the user, such that said user can take remedying actions, such as to clean the appliance interface. One such way to communicate the presence of liquid includes notifying the user, e.g. by means of a notification in a graphical user interface of the accessory device, such as the user's smartphone or - watch, or similar device. Such use of an accessory device provides for using the electronic device and the associated electronic system comprising the appliance interface distal the user's attention, meaning that the user is provided with means for being notified of the presence of liquid in the appliance interface, when he/she is not necessarily aware of the electronic device.

Where the electronic device is a monitor device for a medical device, such as a base plate for an ostomy appliance or a wound dressing, and where the electronic system is provided in such medical device, liquid is likely to enter the appliance interface, e.g. due to the user not remembering to decouple the electronic device from the appliance interface in certain situations (e.g. showering or handling the appliance, e.g. in a wet environment, such as in bathroom), or simply not noticing liquid entering the interface. By transmitting a device signal to an accessory device in accordance with determining that liquid is present in the appliance interface, and by visualizing such presence in a user interface of such accessory device, the user is more likely to become aware of the presence of liquid and take remedying actions.

In embodiments, the electronic device is configured to, in accordance with determining that liquid is present in the appliance interface, indicate the presence of liquid by means of a haptic feedback and/or an audio signal. Thus, in embodiments, the electronic device comprises means for notifying/alerting the user, such as through a haptic feedback element and/or a loudspeaker.

In a second aspect of the invention, a method for detecting presence of liquid in an appliance interface is disclosed. The appliance interface comprises at least one primary appliance terminal, a reference appliance terminal, and a dielectric region separating the at least one primary appliance terminal and the reference appliance terminal.

The method comprises the steps of:

- generating and applying an electrical reference signal to the reference appliance terminal,
- detecting an electrical primary signal from the at least one primary appliance terminal,
- comparing the primary signal with the reference signal, and
- determining, in accordance with at least a component of the primary signal being identical to the reference signal, that liquid is present in the appliance interface.

The disclosure pertaining to the appliance interface, and embodiments thereof, according to the first aspect of the invention is considered applicable to the method as disclosed herein.

In embodiments, the method is performed by means of/in an external device, such as in an electronic device according to the first aspect of the invention. The disclosure pertaining to the electronic device, and embodiments thereof, according to the first aspect of the invention is considered applicable to the method as disclosed herein. Thus, embodiments, features, and functionalities disclosed in relation to the first aspect of the invention is likewise applicable to the method according to the second aspect of the invention.

The method as disclosed provides a method for detecting presence of liquid in an appliance interface by means of generating and applying a reference signal to a reference appliance terminal of the appliance interface, detecting a primary signal from at least one primary appliance terminal of the appliance interface and, in accordance with at least a component of the primary signal being identical to the reference signal, determining that liquid is present in the appliance interface. Details on the reference appliance terminal, the at least one primary appliance interface, the appliance interface in general, and the electrical signals are disclosed in relation to the first aspect of the invention.

The step of generating and applying an electrical reference signal to the reference appliance terminal includes generating such reference signal in an external device, such as in an electronic device as previously disclosed. Further, the step of applying a reference signal includes establishing an electrical connection between the external device and the appliance interface, e.g. by means of bringing a terminal of the external device into contact with the reference appliance terminal of the appliance interface. For example, the external device, such as the electronic device, can be configured to couple to the appliance interface in such a way that an electrical (e.g., galvanic) connection between the reference device terminal and the reference appliance terminal is established. In embodiments, the signal is generated by means of a driver or the processor and the power unit, such that the power unit provides the sufficient current or voltage, and the driver or processor generates an appropriate reference signal. In preferred embodiments, the reference signal has a certain characteristic, such as a certain electrical waveform, making it a distinguishable component signal of a possible composite primary signal.

The step of detecting an electrical primary signal from the at least one primary appliance terminal includes establishing an electrical (e.g., galvanic) connection between an external device and the appliance interface, e.g. by means of bringing a terminal of the external device into contact with the primary appliance terminal of the appliance interface. Further, by detecting is meant to obtain certain electrical characteristics, e.g. variances in voltage, current, resistance, or capacitance over time. The primary signal can be a composite signal, such that it can comprise one or more component signals from an appliance (e.g., a sensor assembly) comprising the appliance interface, as was discussed in relation to the first aspect of the invention, and a component signal pertaining to the reference signal. Liquid in the appliance interface can facilitate the conduction of the reference signal from the reference terminal to the at least one primary appliance terminal, and thereby, the reference signal becomes a component signal of the primary signal.

The step of comparing the primary signal with the reference signal includes comparing the two signals and determine their resemblance. In embodiments, the comparison is performed by an external device, e.g. the electronic device, comprising processing means for carrying out such comparison. In embodiments, the step comprises identifying a component signal of the primary signal and comparing such component signal with the reference signal. If at least a component of the primary signal is identical to the reference signal (e.g., if at least a component of the primary signal shares a certain characteristic with the reference signal), it is indicative of liquid in the appliance interface. Because of the presence of (electrically conducting) liquid in the appliance interface, the primary signal, or a component thereof, is identical to the reference signal. In other words, since the reference appliance terminal is (structurally and electrically) separated from the primary appliance terminal by a dielectric region, a resemblance between the reference signal and the primary signal (or component thereof) indicates a presence of liquid: the liquid bridges from the reference appliance terminal to the primary appliance terminal and thereby facilitates the conduction of the reference signal from the reference appliance terminal to the primary appliance terminal, whereby the primary signal (or a component thereof) is identical to the reference signal. In embodiments, resemblance is determined within a certain time period, such as by comparing the past (trailing) 1 second, or other pre-selected time period, of the primary signal with the past (trailing) 1 second, or other pre-selected time period, of the reference signal.

The step of determining that liquid is present in the appliance interface is to be considered conditional on the determination that a component of the primary signal is identical to the reference signal.

In embodiments, by identical is meant that the (component of the) primary signal and the reference signal share a strong resemblance, such as at least 80 %, or at least 90 %, of the measuring points are coinciding. Alternatively, by identical is meant that a frequency of the (component of the) primary signal is identical to a frequency of the reference signal. In embodiments, by identical is meant that a characteristic, such as a waveform, of the primary signal (or a component thereof) resembles the characteristic, such as the waveform, of the reference signal in a qualitative and/or quantitative manner. By a qualitative manner is meant that a person skilled in the art (or an artificially intelligence sufficiently trained in the specific use) is capable of identifying and/or distinguishing the characteristic, such as the waveform, in a composite signal from noise and/or appliance signals (component signals pertaining to an appliance comprising the appliance interface). Thus, the reference signal (i.e., the characteristics, such as the waveform, thereof) is purposively selected to be distinguishable, e.g. by software of the electronic device and/or a skilled person, from appliance signals contained in the (composite) primary signal. For example, the reference signal can be selected by the software of the electronic device, such as through machine learning software considering the appliance signals and, over time, generates a reference signal easily distinguishable from the appliance signals contained in the composite primary signal. Alternatively, the reference signal is selected by the manufacturer of the electronic system, e.g. through the manufacturer's knowledge of the expected appliance signals.

Upon determining that liquid is present in the appliance interface, a system incorporating the method can be configured to generate a notification or to apply a remedying action. For example, a notification can be generated and transmitted to an accessory device, such as a smartphone of a user, the notification prompting such user to clean the interface. Alternatively, the system incorporating the method can be configured to disregard appliance signals until the liquid is removed, i.e. until the appliance interface is dry.

It is foreseen that the method can be incorporated in a variety of systems, including an integral (one-part) system and a two-part system. By an integral (one-part) system is meant that an appliance comprising the appliance interface comprises means for performing the method, including applying an electrical reference signal, detecting an electrical primary signal, comparing the signals, and determining the presence of liquid in the appliance interface. By a two-part system is meant that such system can be considered a plug-and-socket system, wherein one part (e.g., the plug) is a passive part comprising the appliance interface and a second part (e.g., the socket) is an active part comprising the means for performing the method (e.g., the second part is an external device, such as the electronic device as referred to in the first aspect of the invention). By providing an integral system, the presence of liquid in the appliance interface can be monitored continuously regardless of external device(s) to be coupled to the appliance interface. Thus, an integral system can be provided in products/devices such as smartphones, personal devices, or similar, where the presence of liquid in an appliance interface (e.g. a charging port) can severely affect the functionality or lifetime of such products. A two-part system can be provided as one part (e.g., the passive part) being disposable, and the second part (e.g., the active part) being reusable. Thereby is provided a system suitable for disposable appliances, e.g. medical devices, where certain appliances are designed to be for single-use only. For example, where the appliance comprising the appliance interface is a sensor assembly, such sensor assembly can be incorporated in a base plate of an ostomy appliance to provide such base plate with sensing means, or such sensor assembly can be incorporated in a wound dressing to provide monitoring of exudate amounts and propagation. In both cases, a base plate and a wound dressing is commonly designed for single-use only, so it is appreciated that the sensor assembly and the appliance interface thereof is low-cost, i.e. preferably, the passive part does not comprise complicated electronics, e.g. power units and/or processors. By providing a two-part system, the relatively more expensive components configured to carry out the disclosed method, e.g. a power unit and a processor, can be incorporated in a separate (reusable) device, such as the electronic device as disclosed according to the first aspect of the invention.

In an embodiment, the method is performed in an electronic device coupled to the appliance interface.

In embodiments, the electronic device comprises at least one primary device terminal connected to the at least one primary appliance terminal and a reference device terminal connected to the reference appliance terminal. By connected is meant that an electrical (e.g., galvanic) connection is established between the at least one primary device terminal and the at least one primary appliance terminal and between the reference device terminal and the reference appliance terminal. Thereby is provided a two-part (plug-and socket type) system as discussed above. Thereby, the electronics, such as a power unit and a processor, configured to carry out the disclosed method can be included in the electronic device. Thereby, the electronic device can be considered an active part of the system, and the appliance interface can be considered the passive part of the system. In embodiments, the electronic device is reusable, whereas the appliance interface is disposable. Thereby, the appliance interface can be manufactured at a low-cost to provide it as a disposable item, whereas the electronic device can contain the relatively more expensive components.

Embodiments, functionalities, and features of the electronic device, including the at least one primary device terminal and the reference device terminal, as disclosed according to the first aspect of the invention are considered applicable to the present method.

In an embodiment, to generate and apply an electrical reference signal comprises generating an electrical signal having a first electrical waveform.

In embodiments, the first electrical waveform is generated by means of a power unit, e.g. coupled to a processor, of an external device, such as of the electronic device. By a first electrical waveform is meant a waveform having certain characteristics, such as a certain shape, such as a sine waveform, square waveform, triangular waveform or a sawtooth waveform as previously disclosed in relation to the first aspect of the invention.

In embodiments, a machine learning functionality of the electronic device identifies characteristics of the appliance signal(s) over time, such as to generate/provide a reference signal having a waveform being distinguishable from said appliance signal(s). In embodiments, the waveform is selected by the manufacturer. The first electrical waveform is selected such as to provide a distinguishable component signal, such that the reference signal can be identified in the (composite) primary signal, the identification of the reference signal in the primary signal being indicative of liquid in the appliance interface, as previously discussed. Additional details and benefits of providing such a first electrical waveform are disclosed in relation to the first aspect of the invention.

In an embodiment, the appliance interface comprises at least a first primary appliance terminal being a terminal of a first electrode and a second primary appliance terminal being a terminal of a second electrode, and the method comprises the additional steps of:

- applying a voltage across the first primary appliance terminal and the second primary appliance terminal,
- monitoring the voltage across the first primary appliance terminal and the second primary appliance terminal, and
- determining, in accordance with a short-circuit across the first primary appliance terminal and the second primary appliance terminal, that liquid is present across the first electrode and the second electrode.

In embodiments, the first and the second electrode form a sensor for a sensor assembly, the sensor assembly here being the appliance comprising the appliance interface.

In embodiments, the method is performed in an external device, such as in an electronic device as previously disclosed, or the method is performed as part of an integral system.

Applying a voltage to the first primary appliance terminal and the second primary appliance terminal, e.g. by means of a power unit of the external device, consequently applies a voltage across the first electrode and the second electrode comprising the first primary appliance terminal and the second primary appliance terminal, respectively. By monitoring the voltage across the terminals, i.e., over time, certain aspects of the environment surrounding the electrodes can be monitored. In particular, a short-circuit can be indicative of liquid forming a bridge across either the terminals or the electrodes connected thereto. However, by applying different means for detecting liquid in the appliance interface (by comparing (a component of) the primary signal with the reference signal, e.g. by comparing their waveforms), a detected short-circuit can be assigned the electrodes, and hence it is possible to differentiate liquid bridging across electrodes connected to the terminals from liquid bridging across the terminals as such. Thus, by liquid being present across the first electrode and the second electrode is meant that liquid has short-circuited the two electrodes, i.e. liquid forms a (conductive) bridge/path from the first electrode to the second electrode. It should be noted that the described embodiment of the method pertains to liquid detection, but that the method as such can be employed other types of sensors configured to generate a change in voltage based on sensory input (e.g., environmental changes/changes in the surroundings of the sensors).

In embodiments where the electrodes are provided as a sensor of a sensor assembly for a medical device, such as a base plate for an ostomy appliance, the presence of liquid at the electrodes/sensor can be indicative of the presence of output emanating from a stoma. In embodiments, the electronic device is configured to analyze such information and/or to alert a user.

In an embodiment, the method further comprises, in accordance with determining that liquid is present in the appliance interface, the steps of:

- transmitting a device signal to an accessory device comprising a graphical user interface, and
- indicating the presence of liquid in the graphical user interface.

In embodiments, by indicating is meant to visualize, such as to visualize a graphical representation of the appliance interface, or such as to generate a notification, e.g. through a text message or in an app.

By transmitting a device signal to an accessory device in accordance with determining that liquid is present in the appliance interface, and by visualizing such presence in a user interface of such accessory device, the user is more likely to become aware of the presence of liquid and take remedying actions.

In a third aspect of the invention, a substantially planar appliance interface is disclosed. The appliance interface comprises at least one primary appliance terminal, a reference appliance terminal, and a dielectric region separating the at least one primary appliance terminal and the reference appliance terminal. The dielectric region encloses each of the at least one primary appliance terminals.

Embodiments and related definitions disclosed in relation to the appliance interface of the first and/or the second aspect of the invention are considered applicable to the third aspect of the invention.

In embodiments, the appliance interface is substantially planar, such that a surface of the appliance interface is substantially two-dimensional. Thus, the at least one primary appliance terminal, the reference appliance terminal and the dielectric region may be arranged in an appliance interface plane, such that the dielectric region encloses, in the appliance interface plane, each of the at least one primary appliance terminals.

In embodiments, the at least one primary appliance terminal and the reference appliance terminal are provided on a non-conductive substrate and separated from each other, e.g. separated by the dielectric region. In embodiments, the at least one primary appliance terminal is electrically connected to an appliance, such as to at least one electrode of such an appliance. In embodiments, the substrate is stretchable. In embodiments, the substrate is polymeric.

According to the third aspect of the invention, the dielectric region encloses each of the one or more primary appliance terminals. Thus, at least one dielectric region is provided, such as a dielectric region for each of the primary appliance terminals provided. By enclosing is meant that the dielectric region surrounds, in the plane, each of the one or more primary appliance terminals when seen in a direction normal to the appliance interface, i.e. a top view of the appliance interface. In embodiments, the dielectric region extends radially away, in the plane, from an edge of the at least one primary appliance terminal by at least 0.1 mm, such as 0.1 mm, or 0.2 mm, or 0.3 mm, or 0.4 mm, or 0.4 mm, or 0.6 mm, or 0.7 mm, or 0.8 mm, or 0.9 mm, or 1 mm. In embodiments, the dielectric region extends radially away, in the plane, from an edge of the at least one primary appliance terminal by at least 1 mm. In embodiments, the dielectric region extends radially away, in the plane, from an edge of the at least one primary appliance terminal by maximally 5 mm. By providing a maximum distance is facilitated that liquid present in the appliance interface is likely to bridge across the dielectric region to provide a conduction of an electrical signal from the reference appliance terminal to the primary appliance terminal.

In embodiments, an outer periphery of the dielectric region is adjacent, in the plane, to the reference appliance terminal, such as an enclosing portion of the reference appliance terminal. Thereby, the reference appliance terminal generally covers a substantial (e.g. more than 25 %, or more than 50 %) surface area of the appliance interface. In other words, when providing a substantially planar appliance interface and when seen from a direction normal to said interface (top-view), a dielectric region enclose/surround each of the primary appliance terminal(s), such that said dielectric region separates the at least one primary appliance terminal and the reference appliance terminal. Thus, the (two-dimensional) shape of the reference appliance terminal does not necessarily correspond to the (two-dimensional) shape of the primary appliance terminal. Rather, the reference appliance terminal can comprise a planar contact pad for contacting a corresponding reference device terminal of an electronic device and a planar enclosing portion at least partly enclosing the at least one primary appliance terminal in the plane - separated by a planar dielectric region.

In a fourth aspect of the invention, a medical device comprising the electronic system of the first aspect of the invention is disclosed.

By comprising the electronic system of the first aspect of the invention is meant that the medical device comprises an appliance interface and an electronic device couplable to the appliance interface. In embodiments, the appliance interface is an appliance interface of an appliance embedded/incorporated in the medical device. A medical device comprising an electronic system as previously disclosed provides for a medical device suitable for implementing sensors or similar appliances. For example, the appliance can be embedded or incorporated in the medical device to provide said medical device with the functionalities of the appliance. In embodiments, the appliance is a sensor assembly, and the appliance interface is an appliance interface of such sensor assembly.

Certain medical devices are likely to be exposed to body fluids and/or water or liquids in general. Due to the risk of exposure to body fluids and/or water, it is desired to provide a medical device having an incorporated appliance with the functionalities of the electronic system according to the above aspects of the invention, namely the ability to detect presence of liquid in the appliance interface. Thereby, a user of the medical devices can be notified of such presence of liquid.

In an embodiment, the medical device comprises an ostomy appliance.

By an ostomy appliance is meant at least an appliance comprising a base plate for attachment to a peristomal skin surface of a user having an ostomy by means of an adhesive surface, and optionally a collection bag attached/attachable to the base plate for the collection of output emanating from such ostomy. Thus, according to the embodiment, the ostomy appliance comprises the electronic system according to previous disclosure. In embodiments, the appliance interface is an appliance interface of a sensor assembly for incorporation and/or attachment to the base plate and/or collection bag for sensing various parameters pertaining to the operation of the ostomy appliance, e.g. for alerting the user of an imminent risk of leakage (output escaping the ostomy appliance and becoming a burden to the user).

In embodiments, the sensor assembly is configured for detecting moisture in the adhesive of the base plate and/or for detecting presence of liquid in the interface between the adhesive surface and the peristomal skin surface. In such uses, it is desired to provide the sensor assembly and the appliance interface as a disposable item, such that the user can dispose of the base plate after use without detaching the sensor assembly. Instead, the electronic device can be provided as a reusable item, such that the sensor assembly and the appliance interface can be of relative low cost, whereas the electronic device can comprise relative more expensive electronics, such as the electronics for controlling the appliance. In embodiments, the sensor assembly is provided in a sensor patch for attachment to the proximal adhesive surface of a base plate.

Since an ostomy appliance is configured to be worn at any given time, such as during exercise or showering, the risk of liquid entering the appliance interface is finite. By providing the sensor assembly embedded in the base plate with an electronic system as disclosed, presence of liquid in the appliance interface can be detected. In embodiments, the presence of liquid in the appliance interface can be detected without compromising the functionalities of the sensor assembly.

In an embodiment, the medical device comprises a wound dressing.

By a wound dressing is meant a dressing for covering and/or treating a wound. In embodiments, the wound dressing comprises an absorbent core layer for absorbing exudates from the wound. In embodiments, the wound dressing further comprises a first adhesive layer with a proximal surface for attachment of the wound dressing to the skin surface of a user, and a top-film covering the absorbent core layer. In embodiments, the absorbent core layer comprises a proximal surface, i.e. the skin-facing surface when the user wears the wound dressing, and a distal surface, i.e. the surface facing away from the skin surface. In embodiments, the top-film is arranged on the distal side of the absorbent core layer and the first adhesive layer is arranged on the proximal side of the absorbent core layer.

In embodiments, a sensor assembly - the appliance within the present context - is arranged on the proximal side of the absorbent core layer. In embodiments, the sensor assembly is arranged on the distal side of the absorbent core layer. Providing a wound dressing with a sensor assembly provides for monitoring such wound dressing, such as for monitoring exudate rate, amounts, and patterns. The sensor assembly comprises the appliance interface as disclosed according to aspects of the invention. Thus, the electronic system can be provided as part of a wound dressing, such that the appliance interface and the appliance (sensor assembly) thereof is provided in the (disposable) wound dressing, whereas the electronic device is provided as a reusable device couplable to the wound dressing to provide means for monitoring said wound dressing.

In an embodiment, the medical device comprises a urinary catheter. In embodiments, the urinary catheter is an intermittent urinary catheter.

Providing a urinary catheter with an appliance, such as a sensor assembly for sensing certain characteristics of the (use of the) catheter, may require an external device, such as an electronic device according to aspects of the invention, to be couplable to an appliance interface thereof, such as to provide the appliance along with the catheter as a disposable item. When using a urinary catheter, exposure to body fluids is a factor to consider, since such body fluids potentially affect signals pertaining to the appliance. Thus, by providing the urinary catheter with an electronic system according to aspects of the invention, means for detecting liquid (including body fluids) in an appliance interface of the appliance is provided.

## Detailed description of the drawings

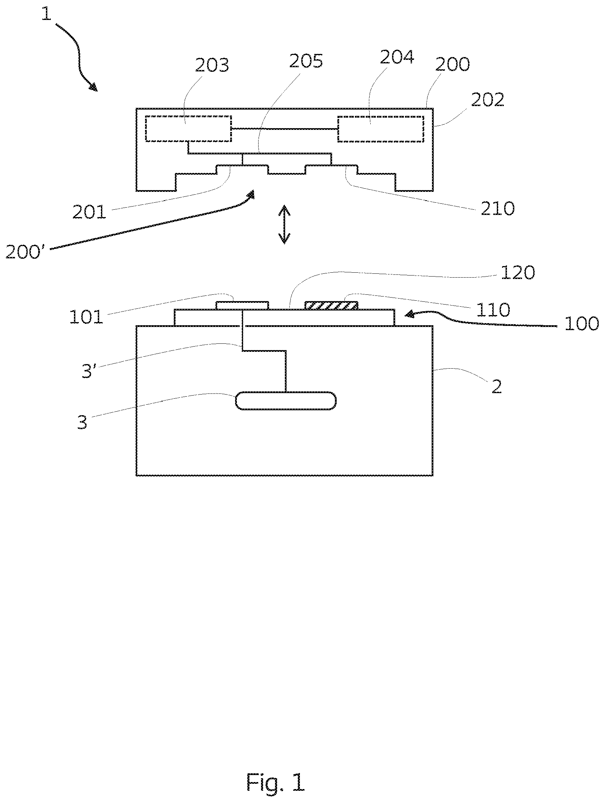

Fig. 1 illustrates an electronic system 1 comprising an electronic device 200 and an appliance interface 100 according to an embodiment of the invention. The appliance interface 100 comprises a primary appliance terminal 101, a reference appliance terminal 110 (hatched for increased intelligibility), and a dielectric region 120 separating the primary appliance terminal 101 and the reference appliance terminal 110. In the illustrated embodiment, the dielectric region 120 is further a substrate for the terminals 101,110. Because of the dielectric region 120, electricity/electrical signals cannot be conducted between the reference appliance terminal 110 and the primary appliance terminal 101 in a dry state of the appliance interface 100. The primary appliance terminal 101 and the reference appliance terminal 110 are conductive, such as made of silver or carbon, such as silver provided, e.g. printed, on the dielectric substrate. The dielectric substrate can be (printable) isobornyl acrylate, but can be anything electrically insulating, including polymers and ceramic materials. By providing printable materials, the entire appliance interface 100, including the terminals 101,110 and the dielectric region 120, can be printed, providing for a fast and cheap production process. In preferred embodiments, the dielectric substrate is stretchable, such as to provide a flexible appliance interface.

The primary appliance terminal 101 is a terminal of an appliance 2 (such as a component, device, or network), such as an appliance 2 comprising a sensor 3. Thereby, data, such as changes in an electrical parameter/quantity, can be extracted from the appliance 2 by means of the electronic device 200 through the primary appliance terminal 101 of the appliance interface 100. Thus, the sensor 3 is electrically connected to the primary appliance terminal 101 through an electrode 3'. The reference appliance terminal 110 is not electrically connected to any part of the appliance 2. Thus, the reference appliance terminal 110 is a conductive terminal solely provided in/on the appliance interface 100.

The electronic device 200 comprises a corresponding device interface 200' comprising a corresponding primary device terminal 201 and a corresponding reference device terminal 210. The electronic device 200 and the appliance interface 100 are configured to engage in such a manner that an electrical (galvanic) connection can be established across the corresponding terminals. According to the illustrated embodiment, the terminals are engageable, whereas in alternative embodiments, the terminals are substantially planar, such that an electrical connection is established by the terminals merely touching each other. According to the illustrated embodiments, the appliance interface 100 resembles a male plug, and the device interface 200' resembles a female socket. In alternative embodiments, the appliance interface 100 resembles a female socket, and the device interface 200' resembles a male plug. In further alternative embodiments, the device interface 200' is configured to receive the appliance interface 100 in a slit, such that the appliance interface is substantially planar and can be inserted into the slit of the electronic device 200. Further engagement options between the appliance interface 100 and the electronic device 200 are foreseen within the scope of the invention, and the illustrated and described embodiments are not intended to limit the claimed invention.

The electronic device 200 comprises a housing 202, a processor 203, a power unit 204 and a circuitry 205. Optionally, the electronic device 200 further comprises a memory (not shown). The power unit 204 can configured to apply a voltage across two or more terminals or to generate an electrical signal. The power unit 204 comprises a battery. The battery can be charged through the device interface 200', such as by connecting the electronic device 200 to a charging unit (not shown) having dimensions corresponding to the appliance interface 100.

The electronic device 200 is configured to apply an electrical reference signal to the reference appliance terminal 110 through the reference device terminal 210, and to detect an electrical primary signal from the primary appliance terminal 101 through the primary device terminal 201. The electronic device 200 is configured to, e.g. by means of the processor 203, compare the primary signal with the reference signal, and, in accordance with at least a component of the primary signal being identical to the reference signal, determine that liquid is present in the appliance interface 100, i.e. in the interface between the appliance interface 100 and the electronic device 200 when these are coupled. By at least a component is meant that the primary signal can be a composite signal comprising one or more component signals (appliance signals) from the appliance 2, and a component signal from the reference appliance terminal 110, i.e. the component carrying the reference signal. Thus, presence of liquid in the appliance interface 100 can be detected while receiving appliance signals from the appliance 2. In other words, in embodiments, the electronic device 200 is configured to identify a component of the primary signal, and, in accordance with such component being identical to the reference signal, determine that liquid is present in the appliance interface 100.

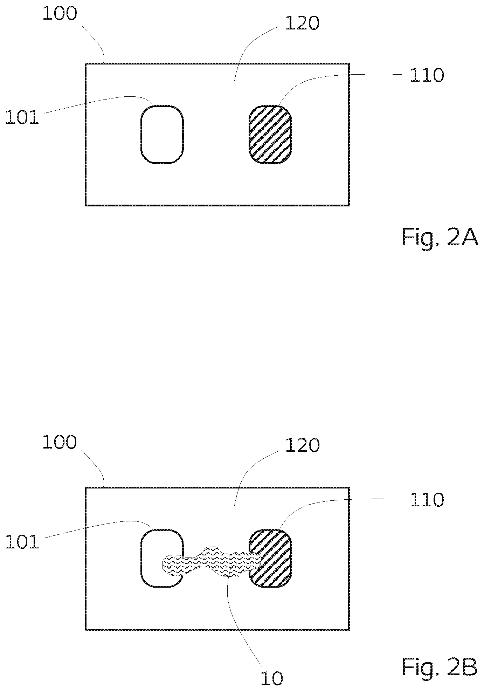

Fig. 2A illustrates a top view of the appliance interface 100 comprising the primary appliance terminal 101 and the reference appliance terminal 110. A dielectric region 120 separates the primary appliance terminal 101 from the reference appliance terminal 110. The appliance interface 100 can be substantially planar, such that the terminals are planar contact pads configured to touch/engage with corresponding contact pads of an electronic device. The primary appliance terminal 101 is in electrical communication with (is a terminal of) an (electrode of an) appliance (not shown), whereas the reference appliance terminal 110 is electrically isolated from any other electrically conductive part.

Fig. 2B illustrates the appliance interface 100 of Fig. 2A, where liquid 10 forms a (conductive) bridge between the reference appliance terminal 110 and the primary appliance terminal 101. Thus, the liquid 10 extends across the dielectric region 120 and hence forms a path for conducting an electrical signal (e.g., the reference signal). When an electronic device 200 configured to apply a reference signal to the reference appliance terminal 110 and to detect a primary signal from the primary appliance terminal 101 is coupled to the appliance interface 100, the reference signal conducts through the liquid 10, whereby (at least a component of) the detected primary signal equals the reference signal, i.e. the signals or at least components thereof are identical. Thus, a determination of at least a component of the primary signal being identical to the reference signal is indicative of the presence of liquid 10 in the appliance interface 100. Upon such determination, the electronic device can be configured to enter a state of safety, whereby damage to sensitive electronics, such as the battery and/or power unit, is avoided. Alternatively, or additionally, upon such determination of the presence of liquid, the electronic device can be configured to alert a user and/or to generate and transmit a device signal to an accessory device, such as a smartphone or smartwatch, indicating the presence of liquid to the user. Further, upon such determination, the electronic device can be configured to disregard appliance signal(s) pertaining to the appliance connected to the primary appliance terminal 101, since such appliance signal(s) can be disrupted and/or faulty because of the presence of the liquid in the appliance interface 100. In embodiments where the appliance interface 100 comprises a plurality of primary appliance terminals, the electronic device can be configured to disregard the appliance signal(s) pertaining to the specific primary appliance terminal in contact with liquid, or to disregard the appliance signals pertaining to all primary appliance terminals, such as until the user has removed the liquid.

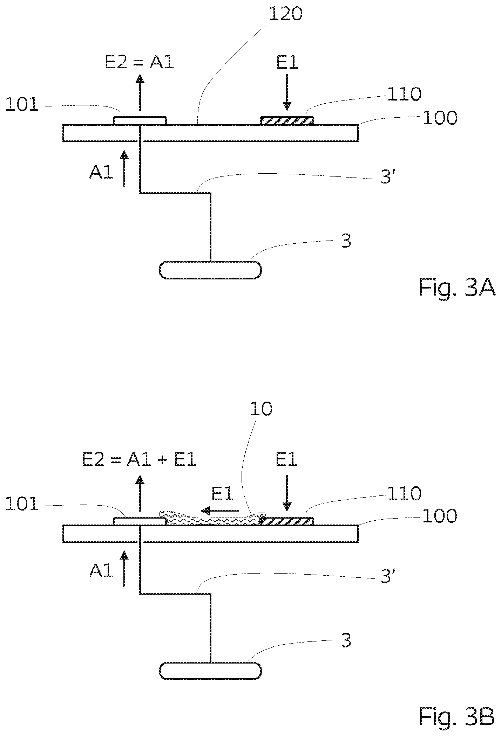

Fig. 3A illustrates an exemplary cross-sectional view of an appliance interface 100 of a sensor 3, i.e., the sensor 3 is the appliance within the present context. The primary appliance terminal 101 is electrically connected to the sensor through the electrode 3'. The electrode 3' as such may form part of the sensor 3. Arrows indicate the transfer of electrical signals. It is appreciated that a more complex situation may arise, such as where the appliance interface comprises multiple appliance terminals or where varying signals are generated, and as such, the indicated transfer of electrical signals are included for schematic and illustrative purposes only. By means of an electronic device (not shown) coupled to the appliance interface 100, a reference signal E1 is applied to the reference appliance terminal 110. Likewise, a primary signal E2 is detected by the electronic device at the primary appliance terminal 101. The primary signal E2 can be a component signal, and in the illustrated case, the primary signal E2 comprises a single component signal: the appliance signal A1. For example, the appliance signal A1 carries information pertaining to the sensor 3.

Fig. 3B illustrates the exemplary cross-sectional view of Fig. 3A in a case where liquid 10 is present on the surface of the appliance interface 100. The liquid 10 forms a conductive path between the reference appliance terminal 110 and the primary appliance terminal 101. Again, arrows indicate the transfer of electrical signals. Due to the presence of liquid 10, the reference signal E1 is conducted through the liquid 10, such that the primary signal E2 detected at the primary appliance terminal 101 is a composite signal comprising two component signals, one being the appliance signal A1, and one being the reference signal E1, such that E2=A1+E1. The electronic device coupled to the appliance interface 100 is configured to compare the primary signal E2 with the reference signal E1. Because a component of the primary signal E2 pertains to the reference signal E1, the electronic device is configured to determine that liquid 10 is present in the appliance interface 100.

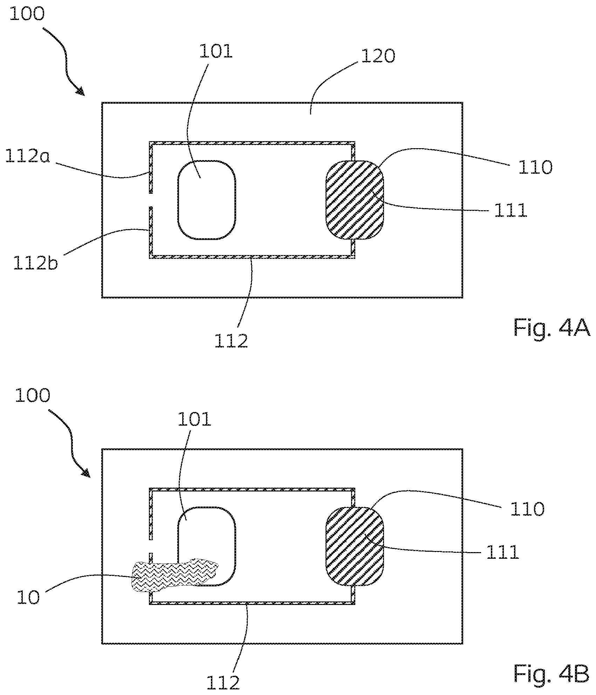

Figs. 4A and 4B illustrate a top view of an appliance interface 100 according to an embodiment of the invention. The appliance interface 100 may be substantially planar and comprises, as in Figs. 2A-2B, a primary appliance terminal 101, a reference appliance terminal 110, and a dielectric region 120 separating the terminals 101,110. The reference appliance terminal 110 comprises a contact pad 111 and an enclosing portion 112. The enclosing portion 112 comprises a first leg 112a and a second leg 112b partly enclosing the primary appliance terminal 101 in the plane of the appliance interface 100. The enclosing portion 112 can be considered an extension of the reference appliance terminal 110, such that liquid 10 can be detected whenever a bridge thereof forms between any part (contact pad 111 and/or enclosing portion 112) of the reference appliance terminal 110 and the primary appliance terminal 101 - see Fig. 4B. The legs 112α,112b of the enclosing portion 112 extends in the same plane as the appliance interface 100, such that by enclosing is meant that the enclosing portion 112 surrounds at least partly the primary appliance terminal 101 in said plane. The enclosing portion 112 increases the likelihood that presence of liquid 10 in the appliance interface 100 is detected according to embodiments of the invention.

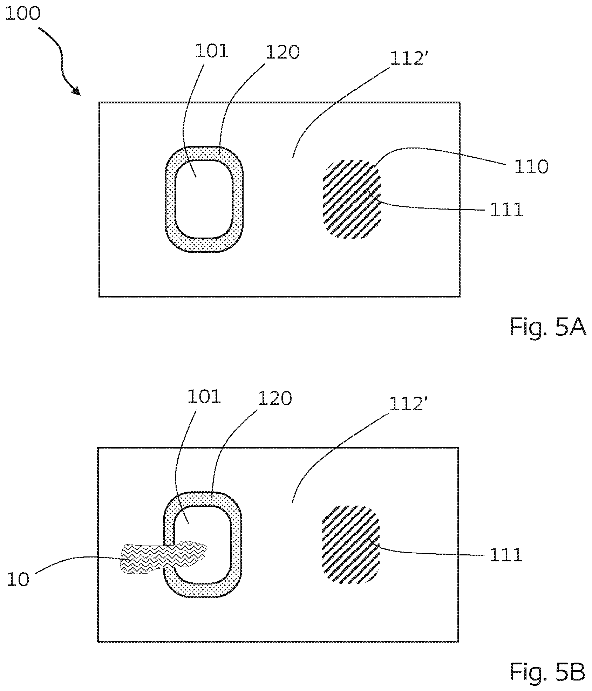

Figs. 5A and 5B illustrate a top view of an appliance interface 100 according to an alternative embodiment of the invention. Here, the dielectric region 120 (dotted) encloses/surrounds the (immediate vicinity of) the primary appliance terminal 101. Thus, the dielectric region 120 extends radially away from a periphery/edge of the primary appliance terminal, e.g. by a distance between 0.1 mm and 2 mm, e.g. a varying distance. The dielectric region 120 electrically insulates the primary appliance terminal 101 from the reference appliance terminal 110. Here, the reference appliance terminal 110 comprises an electrically conductive contact pad 111 and an electrically conductive coherent enclosing portion 112'. Different from the embodiment of Figs. 4A-4B, the enclosing portion 112' is here a coherent/integral body covering a substantial part (e.g., > 50 %) of the surface area of the appliance interface 100. Thus, a substantial part of the surface of the appliance interface 100 is conductive and essentially constitutes the reference appliance terminal to which a reference signal can be applied. However, for practical reasons, a designated area, the contact pad 111, is configured to receive a corresponding reference device terminal of the electronic device. For example, the contact pad 111 can be reinforced to withstand wear and tear resulting from a prolonged galvanic contact with the corresponding reference device terminal. Nevertheless, the contact pad 111 is in electrical communication/integral with the enclosing portion 112'.

Fig. 5B illustrates the situation where liquid 10 forms a bridge across the dielectric region 120 separating the primary appliance terminal 101 and the (enclosing portion 112' of the) reference appliance terminal 110. Here, an electronic device (not shown) coupled to the appliance interface 100 is capable of detecting the presence of liquid 10, since an applied reference signal conducts from the contact pad 111, into the enclosing portion 112', through the liquid 10, and becomes a component signal of the primary signal, as was elaborated in relation to Fig. 3B.

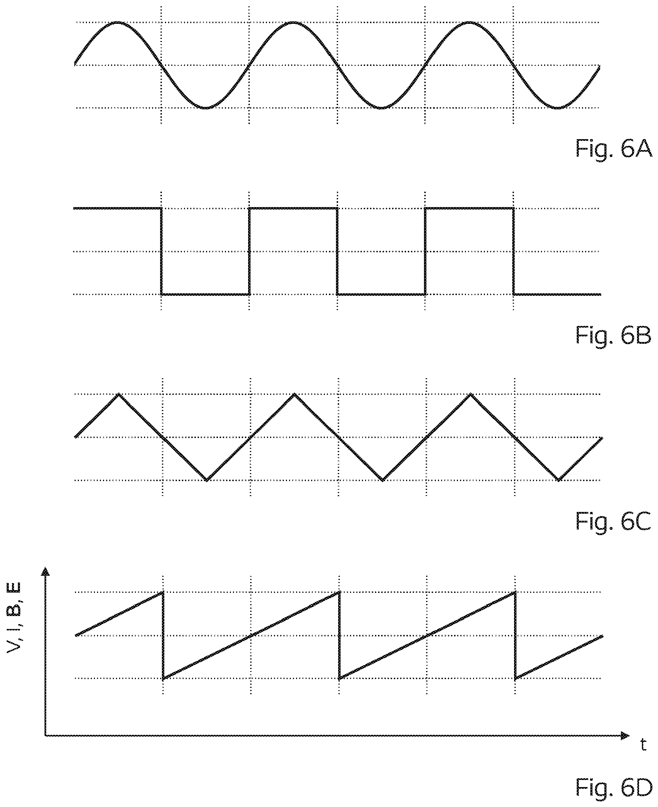

Figs. 6A-6D illustrate four exemplary electrical signals having a certain waveform. According to an aspect of the invention, it is desired to provide a reference signal being distinguishable from appliance signal(s) (in Figs. 3A-3B referred to as A1) pertaining to the appliance, since such appliance signals are contained in the primary signal - potentially along with the reference signal. In other words,, the primary signal can be a composite signal comprising both the appliance signals and the reference signal once said reference signal has been conducted through liquid in the appliance interface.

In particular, since appliance signals can vary according to surroundings (e.g., where the appliance is a sensor) and potentially comprise a degree of noise, it is desired to apply a reference signal to the reference appliance terminal being distinguishable/unique from such expected appliance signals. In embodiments, the electronic device is configured to receive a composite primary signal, i.e. an electrical signal comprising a (component) appliance signal pertaining to the appliance (e.g., sensor) to which the primary appliance terminal is coupled, and - upon presence of liquid in the appliance interface - a component signal pertaining to the reference signal. Thus, when liquid is present in the appliance interface, the electronic device essentially receives a mix/composite of electrical signals in the primary signal, the composite comprising the component signal pertaining to the appliance and the component signal pertaining to the reference signal applied to the reference appliance terminal. When the reference signal is distinguishable from the expected appliance signals, it is possible to identify/distinguish the component signal pertaining to the reference signal from the appliance signals.

One way of providing a distinguishable reference signal is to generate a reference signal having a certain electrical waveform, such as a periodic waveform, which is not expected from the appliance signals. For example, the processor and/or power unit of the electronic device can be configured to generate such certain electrical waveform. For example, the waveform of the reference signal varies (periodically) as voltage V, current I, magnetic field **B**, or electrical field **E**, as a function of time t (see Fig. 6D). In preferred embodiments, the voltage V or current I varies as a function of time t.

Fig. 6A illustrates a sine wave, where the amplitude of the waveform follows a trigonometric sine function with respect to time. Fig. 6B illustrates a square wave of constant period. In alternative embodiments, the period varies, such as to form a rectangular wave. Fig. 6C illustrates a triangular wave. Fig. 6D illustrates a sawtooth wave. The waveforms are included for illustrative purposes only, and the amplitude, wavelength, and/or phase are not necessarily to scale. Alternative waveforms are foreseen within the scope of the invention, as the waveform is merely to be selected to make it distinguishable from the expected appliance signals from the appliance. Thus, in embodiments, the waveform is selected such that it is distinguishable/different from (expected) appliance signals from the appliance. Four such potentially distinguishable waveforms may include a sine wave, a square wave, a triangular wave and a sawtooth wave as illustrated.

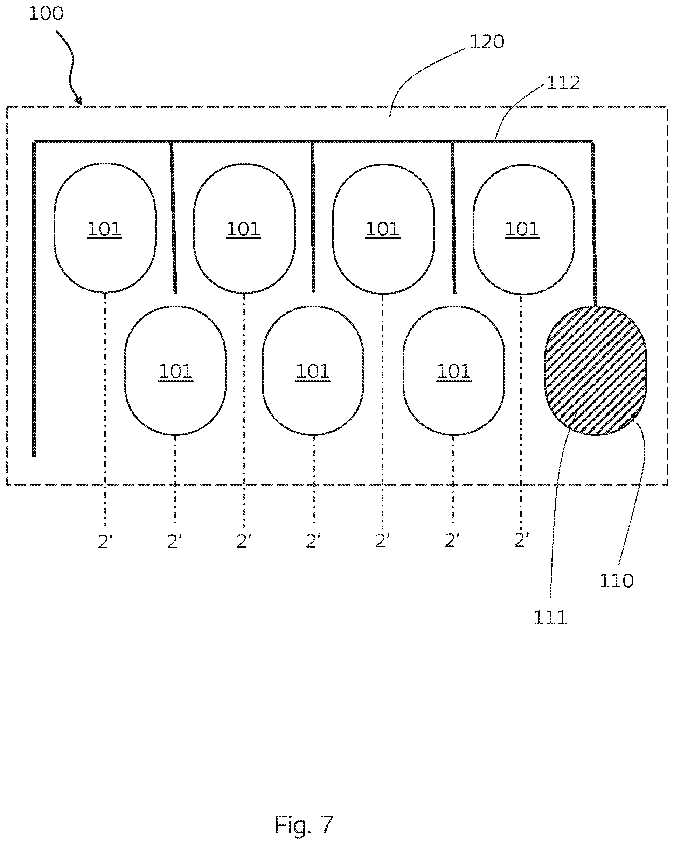

Fig. 7 illustrates a top view of an appliance interface 100 comprising seven primary appliance terminals 101 and a reference appliance terminal 110 according to an embodiment of the invention. Providing a plurality of primary appliance terminals 101 allows for controlling a more complicated appliance, i.e. an appliance electrically connected through electrodes 2' to the primary appliance terminals 101. As previously described, the reference appliance terminal 110 is electrically isolated from the appliance and the electrodes 2' connecting said appliance to the primary appliance terminals 101. The reference appliance terminal 110 of the illustrated embodiment comprises a contact pad 111 and an enclosing portion 112, as previously described. The enclosing portion 112 partly encloses/surrounds, in the plane of the appliance interface 100, the plurality of primary appliance terminals 101 by means of a plurality of legs extending into the gaps between said primary appliance terminals 101. The arrangement and number of legs can vary, such as depending on the arrangement of primary appliance terminals 101 and/or the desired sensitivity towards detecting presence of liquid. Each of the plurality of primary appliance terminals 101 and the contact pad 111 and the enclosing portion 112 are separated by a dielectric region 120, such that an electrical signal can only be conducted between the reference appliance terminal 110 (or the enclosing portion 112 thereof) and one or more of the primary appliance terminals 101 upon presence of liquid in the appliance interface 100.

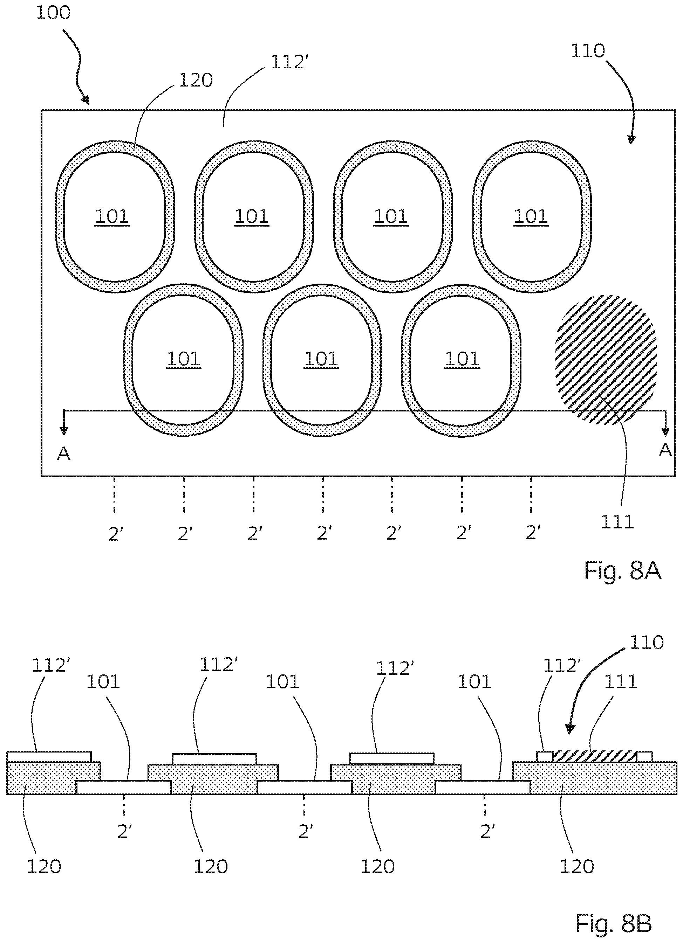

Fig. 8A illustrates an appliance interface 100 comprising seven primary appliance terminals 101 and a reference appliance terminal 110 according to an embodiment of the invention. The appliance interface 100 differs from the one illustrated in Fig. 7 in that the dielectric region 120 (dotted) surrounds the (immediate vicinity of) the primary appliance terminal 101 only (see also Figs. 5A-5B). Further, the reference appliance terminal 110 comprises a contact pad 111 and a coherent enclosing portion 112'. Contrary to the embodiment of Fig. 7, the enclosing portion 112' is a coherent/integral portion covering a substantial part (e.g., > 50 %) of the surface of the appliance interface 100. Thus, a substantial part of the surface of the appliance interface 100 is conductive and essentially constitutes the reference appliance terminal 110 to which a reference signal can be applied by means of an electronic device coupled to the appliance interface 100. However, for practical reasons, a designated area, the contact pad 111, is configured to electrically connect to a corresponding reference device terminal of the electronic device. For example, the contact pad 111 can be reinforced to withstand wear and tear from the contact with the corresponding reference device terminal but is nevertheless in electrical communication/integral with the enclosing portion 112'. The seven primary appliance terminals 101 are coupled to an appliance through the electrodes 2' (the exact arrangement of electrodes 2' may vary but are in general isolated from the reference appliance terminal).

Fig. 8B illustrates a cross-sectional view of the appliance interface of Fig. 8A taken along the line A-A in Fig. 8A. The cross-sectional view illustrates one way of realising the appliance interface 100 of Fig. 8A, but alternative builds of the appliance interface 100 of Fig. 8A are foreseen within the scope of the invention. The cross-sectional view illustrates three primary appliance terminals 101, each connected to an appliance through the electrodes 2'. A dielectric layer forming the dielectric region 120 surrounding each of the primary appliance terminals 101 is arranged (e.g. deposited or printed) between said terminals, and finally, a conductive layer forming the reference appliance terminal 110 (and the enclosing portion 112' thereof) is arranged (e.g. deposited or printed) on top of the dielectric layer. A certain region of the reference appliance terminal 110 constitutes the contact pad 111, whereas the remaining region constitutes the enclosing portion 112'. In embodiments, the dielectric layer forming the dielectric region 120 extends underneath, i.e. supports, the primary appliance terminals 101, such that in alternative ways of realising the appliance interface 100, the dielectric layer 120 is provided as a substrate, and the primary appliance terminals 101 are arranged (e.g. deposited or printed) on top of the dielectric layer 120. Finally, the reference appliance terminal 110 (or enclosing portion 112' thereof) is arranged (e.g. deposited or printed) in between the reference appliance terminals 110, leaving a dielectric region 120 about each of the primary appliance terminals 101. The dimensions are highly exaggerated for illustrative purposes. A thickness of the conductive primary/reference appliance terminals 101, 110 may be within the range of 1 $\mu$m and 50 $\mu$m, such as between 2 $\mu$m and 20 $\mu$m, such as between 4 $\mu$m and 10 $\mu$m.

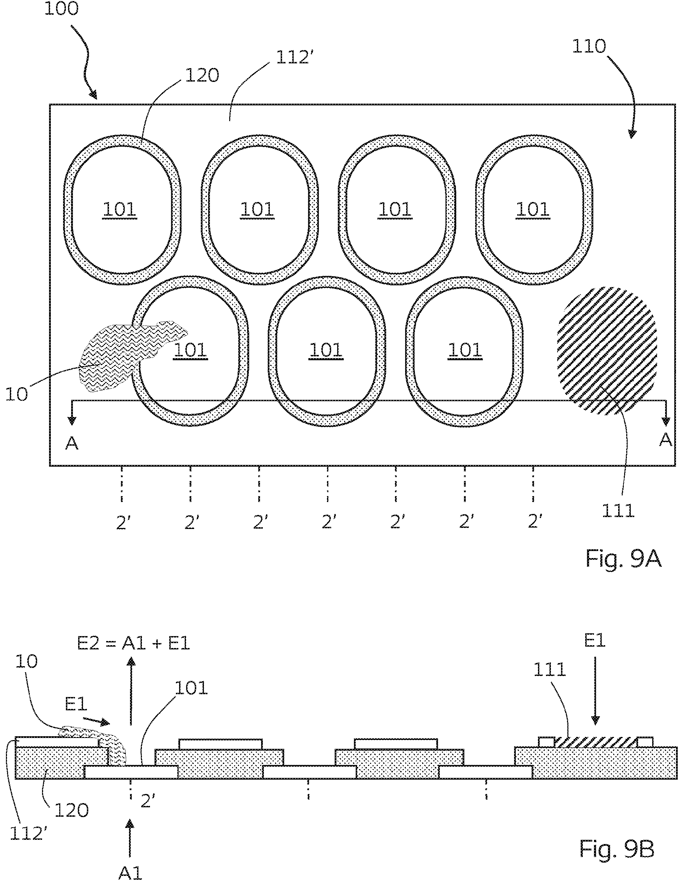

Figs. 9A and 9B illustrate the embodiment of the appliance interface 100 of Figs. 8A-8B, wherein a liquid 10 is present. The liquid 10 extends/forms a bridge from a primary appliance terminal 101 to the enclosing portion 112' of the reference appliance terminal 110. Thereby, a reference signal E1 applied to the reference appliance terminal 110, e.g. through the contact pad 111 and therefrom into the enclosing portion 112', is conducted through the liquid 10 and is detectable in the primary signal E2 at the primary appliance terminal 101 by means of an electronic device coupled to the interface, as previously described. The primary signal E2 can be a composite signal comprising a (component) appliance signal A1, such that the primary signal E2 = A1 + E1. Since the electronic device is configured to compare the primary signal E2 with the reference signal E1 and determine that at least a component (the component pertaining to the reference signal E1) of said primary signal E2 is identical to the reference signal E1, a presence of liquid 10 in the appliance interface 100 can be determined.

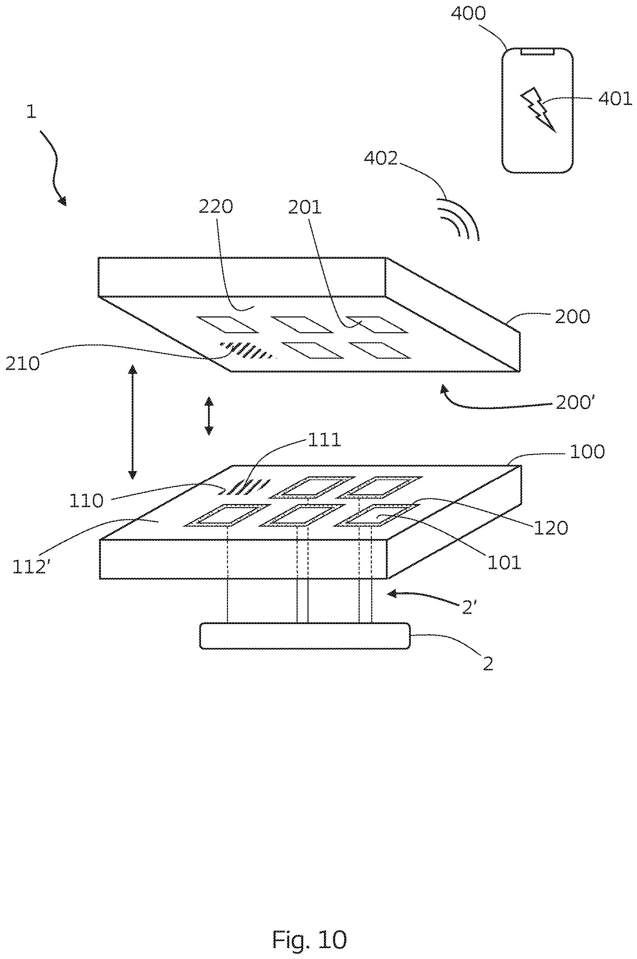

Fig. 10 illustrates a perspective schematic view of an electronic system 1 according to an embodiment of the invention. The electronic system 1 comprises an electronic device 200 and a planar appliance interface 100 couplable to the electronic device 200. The appliance interface 100 comprises the features as previously disclosed; (here, five) primary appliance terminals 101, a reference appliance terminal 110 comprising a contact pad 111 and an enclosing portion 112' enclosing/surrounding the primary appliance terminals 101, and (here, five) dielectric regions 120 (dotted) separating each of the primary appliance terminals 101 from the (enclosing portion 112' of the) reference appliance terminal 110. The appliance interface 100 is an appliance interface of an appliance 2, and the primary appliance terminals 101 are individually electrically connected to an electrode 2' of the appliance 2.

The electronic device 200 comprises a corresponding device interface 200'. The device interface 200' comprises primary device terminals 201 having a size and shape corresponding to the primary appliance terminals 101 and a reference device terminal 210 having a size and shape corresponding to the reference appliance terminal 210. The terminals 201, 210 of the device interface 200' are electrically insulated by a device dielectric region 220.

The electronic device 200 comprises electronics, such as a processor, a power unit and optionally a wireless transceiver configured to transmit device signals 402 wirelessly to an accessory device, such as a smartphone 400 comprising a graphical user interface 401. For example, device signals 402 comprises data pertaining to the appliance 2 or to a determined presence of liquid in the appliance interface 100. Thereby, a user of the electronic system 1 can easily access data through the graphical user interface 401 and act according to generated instructions, such as instructions to clean/dry the appliance interface 100.

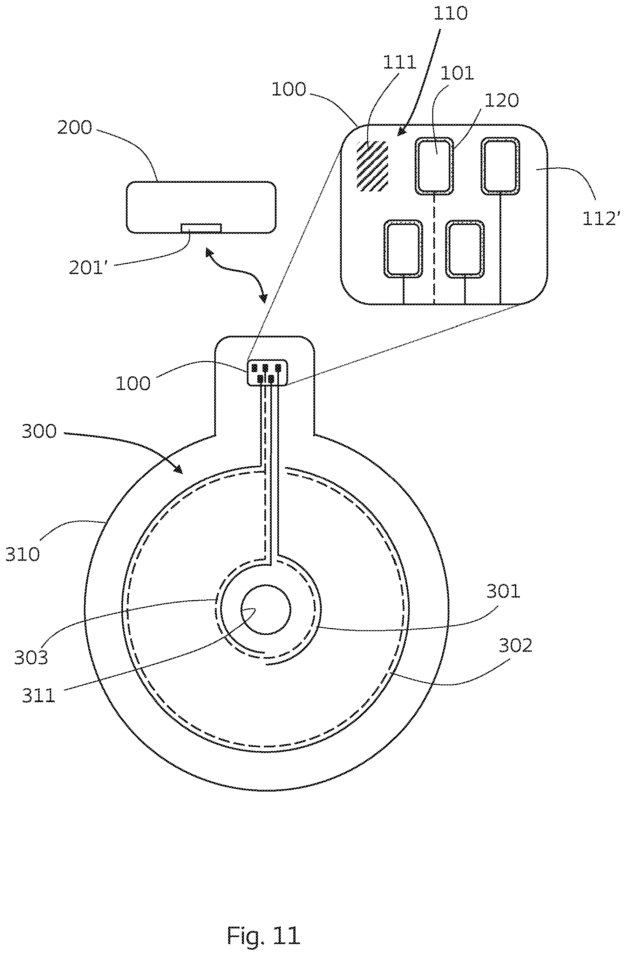

Fig. 11 illustrates an exemplary sensor assembly 300 (appliance) comprising the appliance interface 100 as previously disclosed.

The sensor assembly 300 can be provided in a variety of devices or environments, wherein sensing is desired. For example, the sensor assembly 300 can be provided in a medical device, such as an ostomy appliance or a wound dressing.

For example, the sensor assembly 300 can be provided as part of a base plate 310 of an ostomy appliance, or a sensor patch attachable to a base plate of an ostomy appliance. For example, the sensor assembly 300 can be arranged to facilitate detection of liquid propagating in the interface between an adhesive proximal surface of the base plate 310 and the peristomal skin surface of a user, in order to provide a warning that a leakage of stomal output is imminent.

According to the illustrated embodiment, the sensor assembly 300 comprises three sensors 301,302,303 each comprising two electrodes. The three sensors 301,302,303 are concentric about a stomal opening 311 of the base plate 310. The innermost sensors 301,303 each cover a non-overlapping angle space of 180° about the stomal opening 311. The deashed electrode represents a common ground electrode, such that three sensors 301,302,303 may be formed from four electrodes as illustrated. Different layouts of electrodes may be provided for forming further and/or different sensors, within departing from the scope of the invention.

The electrodes of the sensors 301,302,303 extend into the (planar) appliance interface 100, such that each electrode terminates in a primary appliance terminal 101. An electronic device 200 as previously disclosed is couplable to the appliance interface 100 through the device interface 200', such as to provide means for operating the sensors 301,302,303, such as to detect liquid propagating in the interface between the adhesive proximal surface of the base plate 310 and the peristomal skin surface of the user and/or to detect moisture absorption in the adhesive material of the base plate. Thus, the electronic device 200 can be coupled to the sensor assembly 300 in order to access or gather data from the sensors 301,302,303. For example, the electronic device 200 can be configured to apply a voltage across two electrodes forming a sensor and monitor changes in resistance across the two electrodes, a change in resistance being indicative of moisture absorption in the adhesive base plate and/or presence of liquid in the interface between the adhesive proximal surface of the base plate 310 and the peristomal skin surface of the user. Due to an ostomy appliance being a medical device worn continuously by the user, such as during showering or exercise, an undesired presence of liquid in the appliance interface 100 is likely. However, such undesired presence of a conducting liquid in the appliance interface 100 can cause malfunctioning of the electronic device 200 and/or cause faulty signals from the sensor assembly 300. Thus, it is desired to provide means for detecting presence of such liquid in the appliance interface 100. Such means are provided according to previously disclosed embodiments of the electronic system according to aspects of the invention disclosed herein. According to the embodiment of Fig. 11, the appliance interface 100 comprises four primary appliance terminals 101, each being connected to one of the four electrodes forming the three sensors 301,302,303 of the sensor assembly 300. Each primary appliance terminal 101 is surrounded by a dielectric region 120 separating the primary appliance terminals 101 from the enclosing portion 112' and the contact pad 111 constituting/forming the reference appliance terminal 110. The electrodes are included for illustrative purposes, but it is emphasized that these are isolated from the reference appliance terminal 110, including the enclosing portion 112' thereof. Presence of liquid in the appliance interface 100 is detected by means of the electronic device 200 couplable to the appliance interface 100, as previously described in detail.

When using the appliance interface 100 in a sensor assembly 300 for an ostomy appliance, it is desired to provide the sensor assembly 300, including the appliance interface 100, as a disposable item. Thus, the appliance interface 100 is designed to provide a low manufacturing cost, whereas the electronic device 200 comprises the required electronics needed to operate the appliance (sensor assembly 300) and the appliance interface, such that said electronic device 200 can be reusable and can be coupled to a new appliance interface after use.

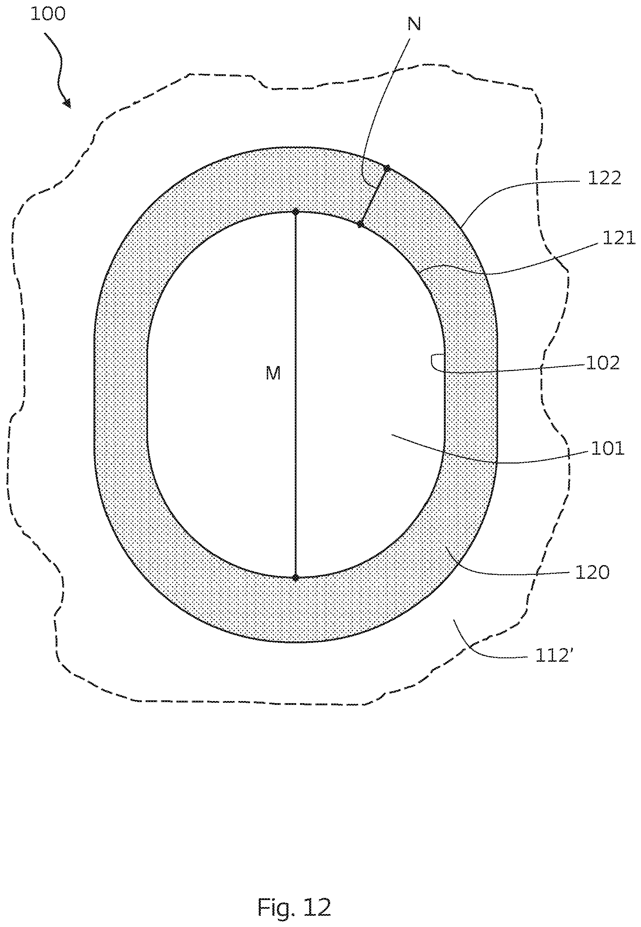

Fig. 12 illustrates a close-up top view of a (planar) appliance interface 100 according to an embodiment of the invention. The appliance interface 100 of Fig. 12 illustrates a primary appliance terminal 101, a dielectric region 120 enclosing/surrounding the primary appliance terminal 101 and a fragment of the enclosing portion 112' (dashed) of the reference appliance terminal. The primary appliance terminal 101 is shaped as a contact pad having certain dimensions. A maximum dimension M is illustrated. In embodiments, the maximum dimension M is between 0.5 mm and 5 mm, or maximally 10 mm. The primary appliance terminal 101 comprises a periphery 102 defining its shape/extent in a two-dimensional geometrical plane. The dielectric region 120 is arranged about the entire periphery 102, such that said dielectric region 120 encloses the primary appliance terminal 101. The dielectric region 120 extends a distance N radially away from the (periphery 102 of) primary appliance terminal 101. For example, the dielectric region 120 extends between 0.1 mm and 2 mm, such as 1 mm radially away from the periphery 102 of the primary appliance terminal 101. The dielectric region 120 separates and insulates the primary appliance terminal 101 from the (enclosing portion 112' of the) reference appliance terminal. Thus, the dielectric region 120 comprises an inner periphery 121 arranged adjacent to the periphery 102 of the primary appliance terminal 101, and an outer periphery 122 arranged adjacent to the enclosing portion 112' of the reference appliance terminal.

Although particular features have been shown and described, it will be understood that they are not intended to limit the claimed invention, and it will be made obvious to those skilled in the art that various changes and modifications may be made without departing from the spirit and scope of the claimed invention. The specification and drawings are, accordingly, to be regarded in an illustrative rather than restrictive sense. The claimed invention is defined by the appended claims.

**Claims**

1. An electronic system (1) for detecting presence of liquid in an appliance interface (100), the system (1) comprising the appliance interface (100) and an electronic device (200) couplable to the appliance interface (100), the appliance interface (100) comprising

    - at least one primary appliance terminal (101),
    - a reference appliance terminal (110), and
    - a dielectric region (120) separating the at least one primary appliance terminal (101) and the reference appliance terminal (110); and
    the electronic device (200) comprising

        - at least one primary device terminal (201) configured to connect to the at least one primary appliance terminal (101), and
        - a reference device terminal (210) configured to connect to the reference appliance terminal (110),

    **characterised in that** the electronic device (200) is configured to

        - generate and apply an electrical reference signal (E1) to the reference appliance terminal (110),
        - detect an electrical primary signal (E2) from the at least one primary appliance terminal (101),
        - compare the primary signal (E2) with the reference signal (E1), and, in accordance with at least a component of the primary signal (E2) being identical to the reference signal (E1), determine that liquid is present in the appliance interface (100).

2. The electronic system according to claim 1, wherein the reference signal has a first electrical waveform.

3. The electronic system according to claim 2, wherein the first electrical waveform is selected from a sine waveform, a square waveform, a rectangular waveform, a triangular waveform, and a saw-toothed waveform.

4. The electronic system according to any one of claims 1-3, wherein the appliance interface is substantially planar.

5. The electronic system according to any one of claims 1-4, wherein the reference appliance terminal (110) comprises a contact pad (111) and an enclosing portion (112), the enclosing portion (112) at least partly enclosing the at least one primary appliance terminal (101).

6. The electronic system according to claim 5, wherein the reference device terminal (210) is configured to connect to the contact pad (111) of the reference appliance terminal (110).

7. The electronic system according to any one of claims 1-6, wherein the appliance interface comprises a first primary appliance terminal and a second primary appliance terminal and wherein the electronic device comprises a first primary device terminal and a second primary device terminal.

8. The electronic system according to claim 7, wherein the electronic system further comprises an appliance (2, 300) connected to the appliance interface (100), and wherein the first primary appliance terminal is a terminal of a first electrode of the appliance, and the second primary appliance terminal is a terminal of a second electrode of the appliance.

9. The electronic system according to any one of claims 7-8, wherein the electronic device is configured to apply a voltage across the first primary appliance terminal and the second primary appliance terminal and configured to detect a short-circuit across the first primary appliance terminal and the second primary appliance terminal.

10. The electronic system according to claim 9 as dependent on claim 8, wherein the electronic device is configured to, in accordance with a detection of a short-circuit across the first primary appliance terminal and the second primary appliance terminal, determine that liquid is present in the appliance (2, 300).

11. The electronic system according to any of claims 1-10, wherein the electronic system further comprises an accessory device (400), and wherein the electronic device (200) is configured to, in accordance with determining that liquid is present in the appliance interface, transmit a device signal to the accessory device (400).

12. The electronic system according to claim 11, wherein the accessory device (400) is configured to, in accordance with receiving the device signal from the electronic device (200), visualize the presence of liquid in α user interface (401) of the accessory device.

13. A method for detecting presence of liquid in an appliance interface (100) comprising at least one primary appliance terminal (101), a reference appliance terminal (110), and a dielectric region (120) separating the at least one primary appliance terminal (101) and the reference appliance terminal (110), **characterised in that** the method comprises the steps of

- generating and applying an electrical reference signal (E1) to the reference appliance terminal (110),
- detecting an electrical primary signal (E2) from the at least one primary appliance terminal,
- comparing the primary signal (E2) with the reference signal (E1), and
- determining, in accordance with at least a component of the primary signal (E2) being identical to the reference signal (E1), that liquid is present in the appliance interface (100).

14. A medical device comprising an electronic system according to any of claims 1-12.

15. The medical device according to claim 14 comprising one of an ostomy appliance, a wound dressing, and a urinary catheter.

**Patentansprüche**

1. Elektronisches System (1) zum Erkennen des Vorhandenseins von Flüssigkeit in einer Vorrichtungsschnittstelle (100), wobei das System (1) die Vorrichtungsschnittstelle (100) und eine mit der Vorrichtungsschnittstelle (100) koppelbare elektronische Einrichtung (200) umfasst, die Vorrichtungsschnittstelle (100) umfassend:

- wenigstens einen primären Vorrichtungsanschluss (101),
- einen Referenzvorrichtungsanschluss (110) und
- einen dielektrischen Bereich (120), der den wenigstens einen primären Vorrichtungsanschluss (101) und den Referenzvorrichtungsanschluss (110) trennt; und
die elektronische Einrichtung (200) umfassend:

- wenigstens einen primären Einrichtungsanschluss (201), der dazu konfiguriert ist, mit dem wenigstens einen primären Vorrichtungsanschluss (101) verbunden zu werden, und
- einen Referenzeinrichtungsanschluss (210), der dazu konfiguriert ist, mit dem Referenzvorrichtungsanschluss (110) verbunden zu werden,

**dadurch gekennzeichnet, dass** die elektronische Einrichtung (200) konfiguriert ist zum

- Erzeugen eines elektrischen Referenzsignal (E1) und Anlegen desselben an den Referenzvorrichtungsanschluss (110),
- Erkennen eines elektrischen primären Signals (E2) von dem wenigstens einen primären Vorrichtungsanschluss (101),
- Vergleichen des primären Signals (E2) mit dem Referenzsignal (E1) und gemäß wenigstens einer Komponente des primären Signals (E2), die mit dem Referenzsignal (E1) identisch ist, Bestimmen, dass Flüssigkeit in der Vorrichtungsschnittstelle (100) vorhanden ist.

2. Elektronisches System nach Anspruch 1, wobei das Referenzsignal eine erste elektrische Wellenform aufweist.

3. Elektronisches System nach Anspruch 2, wobei die erste elektrische Wellenform aus einer Sinuswellenform, einer Quadratwellenform, einer Rechteckwellenform, einer Dreieckswellenform und einer Sägezahnwellenform ausgewählt ist.

4. Elektronisches System nach einem der Ansprüche 1-3, wobei die Vorrichtungsschnittstelle im Wesentlichen planar ist.

5. Elektronisches System nach einem der Ansprüche 1-4, wobei der Referenzvorrichtungsanschluss (110) eine Kontaktfläche (111) und einen umschließenden Abschnitt (112) umfasst, wobei der umschließende Abschnitt (112) den wenigstens einen primären Vorrichtungsanschluss (101) zumindest teilweise umschließt.

6. Elektronisches System nach Anspruch 5, wobei der Referenzeinrichtungsanschluss (210) dazu konfiguriert ist, mit der Kontaktfläche (111) des Referenzvorrichtungsanschlusses (110) verbunden zu werden.

7. Elektronisches System nach einem der Ansprüche 1-6, wobei die Vorrichtungsschnittstelle einen ersten primären Vorrichtungsanschluss und einen zweiten primären Vorrichtungsanschluss umfasst und wobei die elektronische Einrichtung einen ersten primären Einrichtungsanschluss und einen zweiten primären Einrichtungsanschluss umfasst.

8. Elektronisches System nach Anspruch 7, wobei das elektronische System ferner eine Vorrichtung (2, 300) umfasst, die mit der Vorrichtungsschnittstelle (100) verbunden ist, und wobei der erste primäre Vorrichtungsanschluss ein Anschluss einer ersten Elektrode der Vorrichtung ist und der zweite primäre Vorrichtungsanschluss ein Anschluss einer zweiten Elektrode der Vorrichtung ist.

9. Elektronisches System nach einem der Ansprüche 7-8, wobei die elektronische Einrichtung dazu konfiguriert ist, eine Spannung über den ersten primären Vorrichtungsanschluss und den zweiten primären Vorrichtungsanschluss anzulegen, und dazu konfiguriert ist, einen Kurzschluss über dem ersten primären Vorrichtungsanschluss und zweiten primären Vorrichtungsanschluss zu erkennen.

10. Elektronisches System nach Anspruch 9 in Abhängigkeit von Anspruch 8, wobei die elektronische Einrichtung dazu konfiguriert ist, gemäß einer Erkennung eines Kurzschlusses über dem ersten primären Vorrichtungsanschluss und zweiten primären Vorrichtungsanschluss zu bestimmen, dass Flüssigkeit in der Vorrichtung (2, 300) vorhanden ist.

11. Elektronisches System nach einem der Ansprüche 1-10, wobei das elektronische System ferner eine Zubehöreinrichtung (400) umfasst und wobei die elektronische Einrichtung (200) dazu konfiguriert ist, gemäß dem Bestimmen, dass Flüssigkeit in der Vorrichtungsschnittstelle vorhanden ist, ein Einrichtungssignal an die Zubehöreinrichtung (400) zu übertragen.

12. Elektronisches System nach Anspruch 11, wobei die Zubehöreinrichtung (400) dazu konfiguriert ist, gemäß dem Empfangen des Einrichtungssignals von der elektronischen Einrichtung (200) das Vorhandensein von Flüssigkeit in einer Benutzerschnittstelle (401) der Zubehöreinrichtung zu visualisieren.

13. Verfahren zum Erkennen des Vorhandenseins von Flüssigkeit in einer Vorrichtungsschnittstelle (100), die wenigstens einen primären Vorrichtungsanschluss (101), einen Referenzvorrichtungsanschluss (110) und einen dielektrischen Bereich (120), der den wenigstens einen primären Vorrichtungsanschluss (101) und den Referenzvorrichtungsanschluss (110) trennt, umfasst, **dadurch gekennzeichnet, dass** das Verfahren die folgenden Schritte umfasst:

   - Erzeugen und Anlegen eines elektrischen Referenzsignals (E1) an den Referenzvorrichtungsanschluss (110),
   - Erkennen eines elektrischen primären Signals (E2) von dem wenigstens einen primären Vorrichtungsanschluss,
   - Vergleichen des primären Signals (E2) mit dem Referenzsignal (E1), und
   - Bestimmen gemäß wenigstens einer Komponente des primären Signals (E2), die mit dem Referenzsignal (E1) identisch ist, dass Flüssigkeit in der Vorrichtungsschnittstelle (100) vorhanden ist.

14. Medizinische Einrichtung, die ein elektronisches System nach einem der Ansprüche 1-12 umfasst.

15. Medizinische Einrichtung nach Anspruch 14, die eines von einer Ostomievorrichtung, einem Wundverband oder einem Blasenkatheter umfasst.

**Revendications**

1. Système électronique (1) destiné à détecter la présence de liquide dans une interface d'appareil (100), le système (1) comprenant **l'interface d'appareil** (100) et un dispositif électronique (200) pouvant être couplé **à l'interface d'appareil** (100), **l'interface d'appareil** (100) comprenant

   - au moins une borne principale d'appareil (101),
   - une borne de référence d'appareil (110), et
   - une région diélectrique (120) séparant l'au moins une borne principale **d'appareil** (101) et la borne de référence d'appareil (110) ; et
   le dispositif électronique (200) comprenant

   - au moins une borne principale de dispositif (201) conçue pour se brancher à l'au moins une borne principale d'appareil (101), et
   - une borne de référence de dispositif (210) conçue pour se brancher à la borne de référence d'appareil (110),

   **caractérisé en ce que** le dispositif électronique (200) est conçu pour

   - générer et appliquer un signal électrique de référence (E1) à la borne de référence d'appareil (110),
   - détecter un signal électrique principal (E2) provenant de l'au moins une borne principale d'appareil (101),
   - comparer le signal principal (E2) avec le signal de référence (E1) et, si au moins une composante du signal principal (E2) est identique au signal de référence (E1), déterminer que du liquide est présent dans l'interface d'appareil (100).

2. Système électronique selon la revendication 1, dans lequel le signal de référence a une première forme d'onde électrique.

3. Système électronique selon la revendication 2, dans lequel la première forme d'onde électrique est choisie parmi une forme d'onde sinusoïdale, une forme d'onde carrée, une forme d'onde rectangulaire, une forme d'onde triangulaire, et une forme d'onde en dents de scie.

4. Système électronique selon l'une quelconque des revendications 1 à 3, dans lequel l'interface d'appareil est sensiblement plane.

5. Système électronique selon l'une quelconque des revendications 1 à 4, dans lequel la borne de référence d'appareil (110) comprend un plot de contact (111) et une partie enveloppante (112), la partie enveloppante (112) enveloppant au moins partiellement l'au moins une borne principale d'appareil (101).

6. Système électronique selon la revendication 5, dans lequel la borne de référence de dispositif (210) est conçue pour se brancher au plot de contact (111) de la borne de référence d'appareil (110).

7. Système électronique selon l'une quelconque des revendications 1 à 6, dans lequel l'interface d'appareil comprend une première borne principale d'appareil et une deuxième borne principale d'appareil et dans lequel le dispositif électronique comprend une première borne principale de dispositif et une deuxième borne principale de dispositif.

8. Système électronique selon la revendication 7, le système électronique comprenant en outre un appareil (2, 300) branché à l'interface d'appareil (100), et dans lequel la première borne principale d'appareil est une borne d'une première électrode de l'appareil, et la deuxième borne principale d'appareil est une borne d'une deuxième électrode de l'appareil.

9. Système électronique selon l'une quelconque des revendications 7 et 8, dans lequel le dispositif électronique est conçu pour appliquer une tension entre la première borne principale d'appareil et la deuxième borne principale d'appareil et conçu pour détecter un court-circuit entre la première borne principale d'appareil et la deuxième borne principale d'appareil.

10. Système électronique selon la revendication 9 lorsqu'elle est dépendante de la revendication 8, dans lequel le dispositif électronique est conçu pour, en cas de détection d'un court-circuit entre la première borne principale d'appareil et la deuxième borne principale d'appareil, déterminer que du liquide est présent dans l'appareil (2, 300).

**11.** Système électronique selon l'une quelconque des revendications 1 à 10, le système électronique comprenant en outre un dispositif accessoire (400), et dans lequel le dispositif électronique (200) est conçu pour, s'il est déterminé que du liquide est présent dans l'interface d'appareil, transmettre un signal de dispositif au dispositif accessoire (400).

**12.** Système électronique selon la revendication 11, dans lequel le dispositif accessoire (400) est conçu pour, en cas de réception du signal de dispositif depuis le dispositif électronique (200), visualiser la présence de liquide dans une interface utilisateur (401) du dispositif accessoire.

**13.** Procédé destiné à détecter la présence de liquide dans une interface d'appareil (100) comprenant au moins une borne principale d'appareil (101), une borne de référence d'appareil (110), et une région diélectrique (120) séparant l'au moins une borne principale d'appareil (101) et la borne de référence d'appareil (110), **caractérisé en ce que** le procédé comprend les étapes de

- génération et application d'un signal électrique de référence (E1) à la borne de référence d'appareil (110),
- détection d'un signal électrique principal (E2) provenant de l'au moins une borne principale d'appareil,
- comparaison du signal principal (E2) avec le signal de référence (E1), et
- détermination, si au moins une composante du signal principal (E2) est identique au signal de référence (E1), que du liquide est présent dans l'interface d'appareil (100).

**14.** Dispositif médical comprenant un système électronique selon l'une quelconque des revendications 1 à 12.

**15.** Dispositif médical selon la revendication 14 comprenant soit un appareillage de stomie, soit un pansement, soit un cathéter urinaire.

Fig. 1

Fig. 2A

Fig. 2B

120

101

E2 = A1

E1

110

100

A1

3'

3

Fig. 3A

10

101

E2 = A1 + E1

E1

E1

110

100

A1

3'

3

Fig. 3B

Fig. 4A

Fig. 4B

Fig. 5A

Fig. 5B

Fig. 6A

Fig. 6B

Fig. 6C

Fig. 6D

Fig. 7

100

120

112'

110

101 101 101 101

101 101 101

111

A ← ——————————————— → A

2' 2' 2' 2' 2' 2' 2'

Fig. 8A

112' 101 112' 101 112' 101 112' 110
111

2' 2' 2'

120 120 120 120

Fig. 8B

Fig. 9A

Fig. 9B

Fig. 10

Fig. 11

Fig. 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019079037 A1
- WO 2007098762 A1
- US 2009314101 A1